# EUROPEAN PATENT APPLICATION

(11) **EP 2 330 210 A1**
(43) Date of publication of application: **08.06.2011**
(21) Application number: 09810089.4
(22) Date of filing: 01.09.2009
(51) Int. Cl.: C12P 13/00, C12N 15/09, C12P 17/10, C12R 1/465

(54) **PROCESS FOR PRODUCTION OF OPTICALLY ACTIVE AMINE DERIVATIVE**

(30) Priority: 01.09.2008 JP 2008223626
(71) Applicant: Daicel Chemical Industries, Ltd., Osaka-shi, Osaka 530-0001 (JP)
(72) Inventor: NAGASAWA, Toru, Gifu-shi Gifu 501-1174 (JP); YOSHIDA, Toyokazu, Gifu-shi Gifu 501-1121 (JP); ISHIDA, Koichi, Gifu-shi Gifu 501-1131 (JP); YAMAMOTO, Hiroaki, Himeji-shi Hyogo 671-1281 (JP); KIMOTO, Norihiro, Himeji-shi Hyogo 671-1281 (JP)
(74) Representative: Grünecker, Kinkeldey, Stockmair & Schwanhäusser Anwaltssozietät
(86) International application number: PCT/JP2009/065244
(87) International publication number: WO 2010/024445

(57) **Abstract**

Optically active amine derivatives are produced by acting on imine derivatives with a culture of microorganisms having the ability to stereoselectively reduce the compounds, microbial cells, or processed products thereof, followed by collecting the generated optically active amine derivatives. Optically active amine derivatives obtained in the present invention are useful as materials for pharmaceutical agents. The present invention enables, for example, production of an optically active compound represented by formula (IV): 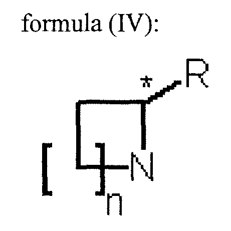 (wherein R group represents an alkyl group having one to three carbon atoms; and n represents an integer of 1 to 4).

SEQUENCE LISTING

<110>  DAICEL CHEMICAL INDUSTRIES, LTD.

<120>  Process for Production of Optically Active Amine Derivative

<130>  EP75225FZapu

<140>  09 810 089.4
<141>  2009-09-01

<150>  PCT/JP2009/065244
<151>  2009-09-01

<150>  JP 2008-223626
<151>  2008-09-01

<160>  12

<170>  PatentIn version 3.4

<210>  1
<211>  1488
<212>  DNA
<213>  Streptomyces sp. GF3546

<400>  1
gagtttgatc ctggctcagg acgaacgctg gcggcgtgct taacacatgc aagtcgaacg      60

atgaagcctt tcggggtgga ttagtggcga acgggtgagt aacacgtggg caatctgccc     120

ttcactctgg gacaagccct ggaaacgggg tctaataccg gataatactt tctcctgcat     180

gggggagggt tgaaagctcc ggcggtgaag gatgagcccg cggcctatca gcttgttggt     240

ggggtgatgg cctaccaagg cgacgacggg tagccggcct gagagggcga ccggccacac     300

tgggactgag acacggccca gactcctacg ggaggcagca gtggggaata ttgcacaatg     360

ggcggaagcc tgatgcagcg acgccgcgtg agggatgacg gccttcgggt tgtaaacctc     420

tttcagcagg gaagaagcgt gagtgacggt acctgcagaa gaagcgccgg ctaactacgt     480

gccagcagcc gcggtaatac gtagggcgca agcgttgtcc ggaattattg ggcgtaaaga     540

gctcgtaggc ggcttgtcgc gtcggatgtg aaagcccggg gcttaacccc gggtctgcat     600

tcgatacggg caggctagag tgtggtaggg gagatcggaa ttcctggtgt agcggtgaaa     660

tgcgcagata tcaggaggaa caccggtggc gaaggcggat ctctgggcca ttactgacgc     720

tgaggagcga aagcgtgggg agcgaacagg attagatacc ctggtagtcc acgccgtaaa     780

cgttgggaac taggtgttgg cgacattcca cgtcgtcggt gccgcagcta acgcattaag     840

ttccccgcct ggggagtacg gccgcaaggc taaaactcaa aggaattgac gggggcccgc     900

acaagcagcg gagcatgtgg cttaattcga cgcaacgcga agaaccttac caaggcttga     960

catacaccgg aaacggccag agatggtcgc ccccttgtgg tcggtgtaca ggtggtgcat    1020

ggctgtcgtc agctcgtgtc gtgagatgtt gggttaagtc ccgcaacgag cgcaaccctt    1080

gttctgtgtt gccagcatgc ctttcggggt gatggggact cacaggagac tgccggggtc    1140

aactcggagg aaggtgggga cgacgtcaag tcatcatgcc ccttatgtct tgggctgcac    1200

acgtgctaca atggccggta caatgagctg cgatgccgtg aggcggagcg aatctcaaaa    1260

agccggtctc agttcggatt ggggtctgca actcgacccc atgaagtcgg agttgctagt 1320

aatcgcagat cagcattgct gcggtgaata cgttcccggg ccttgtacac accgcccgtc 1380

acgtcacgaa agtcggtaac acccgaagcc ggtggcccaa cccccttgtg gggagggagc 1440

tgtcgaaggt gggactggcg attgggacga agtcgtaaca aggtagcc 1488

<210> 2
<211> 873
<212> DNA
<213> Streptomyces sp. GF 3546

<400> 2
atgagcaagc agtcggtaac ggtcatcggt ctgggcccga tgggccaggc gatggtgaac 60

accttcctgg acaacggcca cgaggtgacc gtgtggaacc ggaccgccag caaggccgag 120

gcgctcgtgg cccggggtgc cgtactggct cccaccgtcg aggacgcgct cagcgccaat 180

gagctgatcg tgctcagcct gacggactac gacgcggtgt acgccattct tgagccggtg 240

acgggttccc tgtccggcaa ggtcatcgcc aacctcagct ccgacacccc ggacaaggcc 300

cgcgaggcgg ccaagtgggc ggccaagcac ggtgcgaagc acctcaccgg tggtgtgcag 360

gtgccgccgc cgctgatcgg caagcccgag tcctccacct actacagcgg tcccaaggat 420

gtcttcgacg cccatgagga caccctgaag gtcctcacca acgcggacta ccgcggcgag 480

gacgccggcc tcgcggccat gtactaccag gcccagatga ccatcttctg gaccacgatg 540

ctgagctact accagaccct cgcgctgggc caggccaacg gtgtctcggc gaaggaactg 600

ctgccctacg ccacgatgat gacgtcgatg atgccgcact tcctggagct gtacgcccag 660

cacgtggact ccgcggacta cccgggcgac gtggaccggc tcgcgatggg ggcggccagt 720

gtcgaccacg tcctgcacac gcaccaggac gcgggcgtca gcaccgtgct gccggccgcc 780

gtcgccgaga tcttcaaggc gggcatggag aagggcttcg ccgagaacag cttctccagc 840

ctcatcgagg tgctcaagaa gccggcggtc tga 873

<210> 3
<211> 290
<212> PRT
<213> Streptomyces sp. GF 3546

<400> 3

Met Ser Lys Gln Ser Val Thr Val Ile Gly Leu Gly Pro Met Gly Gln
1               5                   10                  15

Ala Met Val Asn Thr Phe Leu Asp Asn Gly His Glu Val Thr Val Trp
            20                  25                  30

Asn Arg Thr Ala Ser Lys Ala Glu Ala Leu Val Ala Arg Gly Ala Val
        35                  40                  45

Leu Ala Pro Thr Val Glu Asp Ala Leu Ser Ala Asn Glu Leu Ile Val
    50                  55                  60

Leu Ser Leu Thr Asp Tyr Asp Ala Val Tyr Ala Ile Leu Glu Pro Val
65                  70                  75                  80

Thr Gly Ser Leu Ser Gly Lys Val Ile Ala Asn Leu Ser Ser Asp Thr
                85                  90                  95

Pro Asp Lys Ala Arg Glu Ala Ala Lys Trp Ala Ala Lys His Gly Ala
            100                 105                 110

Lys His Leu Thr Gly Gly Val Gln Val Pro Pro Pro Leu Ile Gly Lys
        115                 120                 125

Pro Glu Ser Ser Thr Tyr Tyr Ser Gly Pro Lys Asp Val Phe Asp Ala
    130                 135                 140

His Glu Asp Thr Leu Lys Val Leu Thr Asn Ala Asp Tyr Arg Gly Glu
145                 150                 155                 160

Asp Ala Gly Leu Ala Ala Met Tyr Tyr Gln Ala Gln Met Thr Ile Phe
                165                 170                 175

Trp Thr Thr Met Leu Ser Tyr Tyr Gln Thr Leu Ala Leu Gly Gln Ala
            180                 185                 190

Asn Gly Val Ser Ala Lys Glu Leu Leu Pro Tyr Ala Thr Met Met Thr
        195                 200                 205

Ser Met Met Pro His Phe Leu Glu Leu Tyr Ala Gln His Val Asp Ser
    210                 215                 220

Ala Asp Tyr Pro Gly Asp Val Asp Arg Leu Ala Met Gly Ala Ala Ser
225                 230                 235                 240

Val Asp His Val Leu His Thr His Gln Asp Ala Gly Val Ser Thr Val
                245                 250                 255

Leu Pro Ala Ala Val Ala Glu Ile Phe Lys Ala Gly Met Glu Lys Gly
            260                 265                 270

Phe Ala Glu Asn Ser Phe Ser Ser Leu Ile Glu Val Leu Lys Lys Pro
        275                 280                 285

Ala Val
    290

<210>  4
<211>  12
<212>  PRT
<213>  Streptomyces sp. GF 3546

<400>  4

Ser Lys Gln Ser Val Thr Val Ile Gly Leu Gly Pro
1               5                   10

<210>  5
<211>  12
<212>  PRT
<213>  Streptomyces sp. GF 3546

<400>  5

Gly Phe Ala Glu Asn Ser Phe Ser Ser Leu Ile Glu
1               5                   10

<210>  6
<211>  35
<212>  DNA
<213>  Artificial

<220>
<223>  An artificially synthesized primer sequence

<400>  6
gacgaattct awssaagcag wssgtsacsg tsatc                                  35

<210>  7
<211>  35
<212>  DNA
<213>  Artificial

<220>
<223>  An artificially synthesized primer sequence

<400>  7
gacgaattct agatsarssw sswgaagctg ttytc                                  35

<210>  8
<211>  800
<212>  DNA
<213>  Streptomyces sp. GF 3546

<400>  8
atcggtctgg gcccgatggg ccaggcgatg gtgaacacct tcctggacaa cggccacgag      60

gtgaccgtgt ggaaccggac cgccagcaag gccgaggcgc tcgtggcccg gggtgccgta     120

ctggctccca ccgtcgagga cgcgctcagc gccaatgagc tgatcgtgct cagcctgacg     180

gactacgacg cggtgtacgc cattcttgag ccggtgacgg gttccctgtc cggcaaggtc     240

atcgccaacc tcagctccga caccccggac aaggcccgcg aggcggccaa gtgggcggcc     300

aagcacggtg cgaagcacct caccggtggt gtgcaggtgc cgccgccgct gatcggcaag     360

cccgagtcct ccacctacta cagcggtccc aaggatgtct tcgacgccca tgaggacacc     420

ctgaaggtcc tcaccaacgc ggactaccgc ggcgaggacg ccggcctcgc ggccatgtac     480

taccaggccc agatgaccat cttctggacc acgatgctga gctactacca gaccctcgcg     540

ctgggccagg ccaacggtgt ctcggcgaag gaactgctgc cctacgccac gatgatgacg     600

tcgatgatgc cgcacttcct ggagctgtac gcccagcacg tggactccgc ggactacccg     660

ggcgacgtgg accggctcgc gatgggggcg gccagtgtcg accacgtcct gcacacgcac 720

caggacgcgg gcgtcagcac cgtgctgccg gccgccgtcg ccgagatctt caaggcgggc 780

atggagaagg gcttcgccga 800

<210> 9
<211> 21
<212> DNA
<213> Artificial

<220>
<223> An artificially synthesized primer sequence

<400> 9
gctgtagtag gtggaggact c 21

<210> 10
<211> 20
<212> DNA
<213> Artificial

<220>
<223> An artificially synthesized primer sequence

<400> 10
tacgcccagc acgtggactc 20

<210> 11
<211> 32
<212> DNA
<213> Artificial

<220>
<223> An artificially synthesized primer sequence

<400> 11
cattcatgag taaacaatcg gtaacggtca tc 32

<210> 12
<211> 33
<212> DNA
<213> Artificial

<220>
<223> An artificially synthesized primer sequence

<400> 12
ctgtctagat tatacagcag gcttcttgag cac 33

## Description

### Technical Field

The present invention relates to methods for producing optically active amine derivatives to be used as raw materials or intermediates for synthesizing various pharmaceuticals by stereoselectively reducing imine derivatives.

### Background Art

Other than the report listed below, little is known about methods for producing optically active amine derivatives by stereoselectively reducing imine derivatives.
Non-patent Document 1: Tetrahedron Asymmetry, 19, 93-96, 2008.

### Reduction of N-phenyl-1-phenylethylamine derivatives by Candida parapsilosis

However, since the reduced product of N-benzylidenebenzylamine has no chirality, the stereoselectivity of the reaction is unclear. Furthermore, the reduction of N-((E)-N-(1-phenylethylidene) aniline derivatives by *Candida parapsilosis* is very inefficient and thus is not suitable for industrial application. In addition, the enzymes responsible for the imine reduction remain unidentified.

On the other hand, the following methods are known for producing 2-methylpyrrolidine:
Non-patent Document 2: Acta. Pharm. Suec., 15, 255-263, 1978.

Method for producing chiral 2-methylpyrrolidine by optically resolving racemic 2-methylpyrrolidine using tartaric acid, in which racemic 2-methylpyrrolidine is produced by reducing 2-methyl-1-pyrroline with sodium borohydride.
Non-patent Document 3: J. Org. Chem., 54, 209-216, 1989.

Method for producing 2-methylpyrrolidine, in which the hydroxyl group of L-prolinol derived from L-proline is converted into a chloro group using thionyl chloride; the nitrogen atom is protected with a benzyloxycarbonyl group; and then
(R)-1-benzyloxycarbonyl-2-methylpyrrolidine is produced via radical-based reduction using tributyltin hydride, followed by deprotection.
Patent Document 1: WO2005/073388

Method for producing 2-methylpyrrolidine, in which optically active 1,4-pentanediol is converted into disulfonate and cyclized by reaction with amine.

However, all of these methods have the following problems:
- the process is long;
- dangerous hydride reagents are used;
- and the yield does not exceed 50% since the product is produced by a resolution method

### [Prior Art Documents]

### [Non-patent Documents]

[Non-patent Document 1] Tetrahedron Asymmetry, 19, 93-96 (2008)
[Non-patent Document 2] Acta. Pharm. Suec., 15, 255-263 (1978)
[Non-patent Document 3] J. Org. Chem., 54, 209-216 (1989)

### [Patent Documents]

[Patent Document 1] WO2005/073388

### Disclosure of the Invention

### [Problems to be Solved by the Invention]

An objective of the present invention is to provide methods for producing optically active amine derivatives from imine derivatives. Another objective of the present invention is to provide microorganisms and enzymes that are able to produce optically active amine derivatives using imine derivatives as substrates. Still another objective of the present invention is to obtain recombinants by isolating DNAs encoding enzymes having the properties of interest. Yet another objective of the present invention is to provide methods for producing optically active amine derivatives using the recombinants.

### [Means for Solving the Problems]

The present inventors conducted dedicated studies to find a method that meets such advanced requirements. As a result, the present inventors discovered that optically active amine derivatives could be produced by stereoselectively reducing imine derivatives using the microorganism's ability to reduce stereoselectively.

As described above, there are known enzymes involved in biosynthesis that stereoselectively reduce imine derivatives. However, all of the known enzymes are involved in metabolism. Therefore, substrate specificity and activity required for industrial application cannot be expected when the enzymes are used to produce intermediates for pharmaceuticals or such. In particular, there is no available method for producing optically active cyclic alkyl amine derivatives by reducing cyclic alkyl imine derivatives such as 2-methyl-1-pyrroline.

Thus, it was a very surprising result to discover microorganisms that have substrate specificity and catalytic activity suitable for production of intermediates for pharmaceuticals or the like. More surprisingly, the ability to stereoselectively reduce imines of the microorganisms was sufficiently high and meets the requirements of industrial application. For example, *Streptomyces* sp. GF3546 was revealed to be a microorganism that has useful properties to solve the above-mentioned objectives. The present inventors also successfully purified the imine reductase by preparing cell-free extracts from a large-scale culture of GF3546, followed by the steps described below:
- hydrophobic chromatography using Phenylsepharose; and
- ion exchange chromatography using MonoQ.

Furthermore, the present inventors isolated a DNA encoding the imine reductase and then produced recombinant bacteria expressing the enzyme at high levels, thereby completing the present invention.

Specifically, the present invention provides methods for producing optically active amines described below. The present invention also relates to microorganisms and imine reductases that are useful for the methods, polynucleotides comprising DNAs encoding such enzymes, methods for producing the enzymes.
[1] An imine reductase having the physicochemical properties of (1) to (5) below:
   (1) activity: to generate (S)-2-methylpyrrolidine by reducing 2-methyl-1-pyrroline in a reduced nicotinamide adenine dinucleotide phosphate (hereinafter abbreviated as NADPH)-dependent manner and generate 2-methyl-1-pyrroline by oxidizing (S)-2-methylpyrrolidine in a nicotinamide adenine dinucleotide phosphate (hereinafter abbreviated as NADP⁺)-dependent manner;
   (2) coenzyme dependency: use NADPH as a coenzyme for reduction and use NADP⁺ as a coenzyme for oxidation;
   (3) optimal pH: 7.4 to 8.0 for reduction and pH 10.5 for oxidation;
   (4) optimal temperature: 35 to 45°C for reduction; and
   (5) molecular weight: approximately 30,500 by sodium dodecyl sulfate-polyacrylamide gel electrophoresis (hereinafter abbreviated as SDS-PAGE) and approximately 81,500 by gel filtration;
[2] an imine reductase that has the physicochemical properties of (1) and (2) of [1], and which is encoded by the polynucleotide of any one of (a) to (e) below:
   (a) a polynucleotide comprising the nucleotide sequence of SEQ ID NO: 2;
   (b) a polynucleotide encoding a protein comprising the amino acid sequence of SEQ ID NO: 3;
   (c) a polynucleotide encoding a protein comprising an amino acid sequence with a substitution, deletion, insertion, and/or addition of one or more amino acids in the amino acid sequence of SEQ ID NO: 3;
   (d) a polynucleotide that hybridizes under a stringent condition to a DNA comprising the nucleotide sequence of SEQ ID NO: 2; and
   (e) a polynucleotide encoding an amino acid sequence with 80% or higher sequence identity to the amino acid sequence of SEQ ID NO: 3;
[3] a method for producing an optically active S-isomer amine derivative, which comprises the steps of:
   contacting an imine derivative represented by formula (I) with at least one enzymatically active material selected from the group consisting of: the imine reductase of [1] or [2], a microbial cell that produces the imine reductase, and a processed product thereof; and collecting the optically active S-isomer amine derivative represented by formula (II): 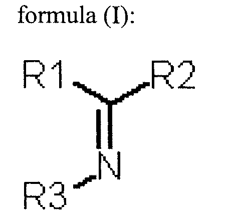 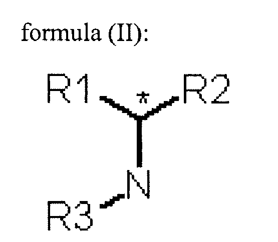 (wherein R1 and R2 groups each represents an alkyl group having one to three carbon atoms; R3 group represents a hydrogen or an alkyl group having one to three carbon atoms; and R1 and R3 together may form a ring);
[4] a method for producing an optically active S-isomer amine derivative, which comprises the steps of:
   contacting an imine derivative represented by formula (I) with at least one enzymatically active material selected from the group consisting of: a culture of microorganism having the ability to stereoselectively reduce the compound, a microbial cell, and a processed product thereof; and
   collecting the optically active S-isomer amine derivative represented by formula (II): 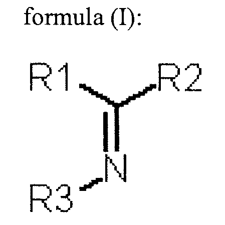 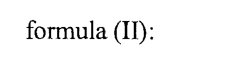 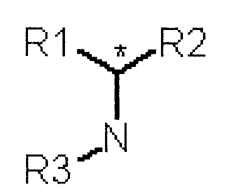
   (wherein R1 and R2 groups each represents an alkyl group having one to three carbon atoms; R3 group represents a hydrogen or an alkyl group having one to three carbon atoms; and R1 and R3 together may form a ring);
[5] the method of [1], wherein the compound of formula (I) is an imine derivative represented by formula (III) below and the compound of formula (II) is an amine derivative represented by formula (IV): 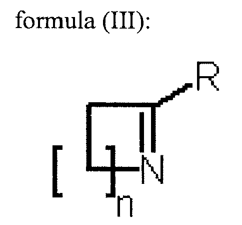 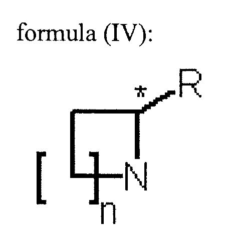 (wherein R group represents an alkyl group having one to three carbon atoms; and n represents an integer of 1 to 4);
[6] the method of [5] for producing an optically active S-isomer amine derivative, wherein the compound represented by formula (III) is 2-methyl-1-pyrroline and the compound represented by formula (IV) is 2-methylpyrrolidine;
[7] the method of any one of [4] to [6] for producing an optically active S-isomer amine derivative, wherein the microorganism belongs to the genus *Streptomyces*;
[8] the method of any one of [4] to [6] for producing an optically active S-isomer amine derivative, wherein the microorganism is *Streptomyces* sp;
[9] the method of any one of [4] to [6] for producing an optically active S-isomer amine derivative, wherein the microorganism is *Streptomyces* sp. NITE P-593;
[10] a polynucleotide encoding an imine reductase that has the physicochemical properties of (1) and (2) of [1], which is any one of the following polynucleotides:
   (a) a polynucleotide comprising the nucleotide sequence of SEQ ID NO: 2;
   (b) a polynucleotide encoding a protein comprising the amino acid sequence of SEQ ID NO: 3;
   (c) a polynucleotide encoding a protein comprising an amino acid sequence with a substitution, deletion, insertion, and/or addition of one or more amino acids in the amino acid sequence of SEQ ID NO: 3;
   (d) a polynucleotide that hybridizes under a stringent condition to a DNA comprising the nucleotide sequence of SEQ ID NO: 2; and
   (e) a polynucleotide encoding an amino acid sequence with 80% or higher sequence identity to the amino acid sequence of SEQ ID NO: 3;
[11] a recombinant vector inserted with the polynucleotide of [10];
[12] the recombinant vector of [11], which is inserted additionally with a polynucleotide encoding a dehydrogenase that is capable of catalyzing a redox reaction using NADP⁺ as a coenzyme;
[13] the vector of [12], wherein the dehydrogenase is a glucose dehydrogenase;
[14] the recombinant vector of [12], wherein the glucose dehydrogenase is derived from *Bacillus subtilis*;
[15] a transformant that maintains the polynucleotide of [10] or the vector of [12] in an expressible manner; and
[16] a method for producing a protein encoded by the polynucleotide of [10], which comprises the step of culturing the transformant of [15] and collecting an expression product.

### [Effects of the Invention]

The present invention provides methods for efficiently producing optically active amine (S) isomers by stereoselectively reducing imines using the reducing ability of microorganisms. The present invention also provides imine reductases that catalyze the reaction. In addition, the present invention provides recombinant bacteria expressing the enzymes at high levels by isolating DNAs encoding the above-described imine reductases. The methods of the present invention which use the high stereoselectivity of a microorganism are industrially advantageous because optically active amines can be synthesized using as a substrate an imine derivative that can be synthesized at low cost. The present invention is particularly useful in producing (S)-2-methylpyrrolidine. (S)-2-Methylpyrrolidine is a useful compound as an intermediate in pharmaceutical synthesis. The present invention enables simple, efficient production of the compound by using the activity of microorganisms or enzymes.

### Brief Description of the Drawings

Fig. 1 is a diagram showing a molecular phylogenetic tree constructed based on the nucleotide sequence of 16S rDNA (SEQ ID NO: 1) of *Streptomyces* sp. GF3546.
Fig. 2 is a graph showing a result of 2-methyl-1-pyrroline (2-MPN) reduction by *Streptomyces* sp. GF3546. The vertical axis indicates the amount (mM) of (S)-2-methylpyrrolidine produced, while the horizontal axis indicates reaction time (hour).
Fig. 3 is a graph showing a result of assessing the optimal pH (reduction) of imine reductase derived from *Streptomyces* sp. GF3546. The vertical axis indicates the relative activity (%) when the maximal activity is taken as 100, while the horizontal axis indicates the pH of enzyme reaction mixture.
Fig.4 is a graph showing a result of assessing the optimal pH (oxidation) of imine reductase derived from *Streptomyces* sp. GF3546. The vertical axis indicates the relative activity (%) when the maximal activity is taken as 100, while the horizontal axis indicates the pH of enzyme reaction mixture.
Fig. 5 is a graph showing a result of assessing the optimal temperature (reduction) of imine reductase derived from *Streptomyces* sp. GF3546. The vertical axis indicates the relative activity (%) when the maximal activity is taken as 100, while the horizontal axis indicates the temperature (°C) of enzyme reaction mixture.
Fig. 6 is a diagram showing the construction process of plasmid (pSUS2MP1) inserted with the imine reductase gene derived from *Streptomyces* sp. GF3546.
Fig. 7 is a diagram showing the construction process of plasmid (pSG-S2MP) inserted with the *Streptomyces* sp. GF3546-derived imine reductase gene and the Bacillus-derived glucose dehydrogenase gene.

### Mode for Currying Out the Invention

The present invention relates to methods for producing optically active S-isomer amine derivatives, which comprise the steps of:
contacting an imine derivative represented by formula (I) with at least an enzymatically active material selected from the group consisting of a culture of a microorganism having the ability of stereoselectively reducing the compound, the microbial cells, and processed products of the cells;
and collecting the optically active S-isomer amine derivative represented by formula (II). 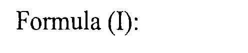 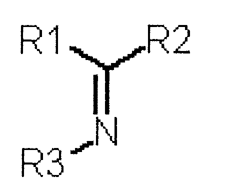 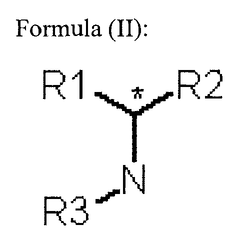
(wherein R1 and R2 groups each represents an alkyl group having one to three carbon atoms; R3 group represents a hydrogen or an alkyl group having one to three carbon atoms; and R1 and R3 may form a ring)

In the present invention, cyclic imine compounds are preferred compounds of formula (I). In the present invention, the cyclic imine compounds include, for example, imine derivatives represented by formula (III) below. 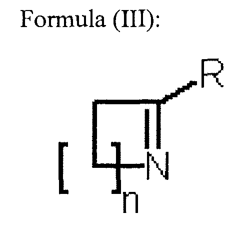 (wherein R group represents an alkyl group having one to three carbon atoms; and n represents an integer of 1 to 4)

The compound represented by formula (IV) as an optically active amine derivative can be produced by using as a substrate a compound represented by formula (III) above. The compound is a preferred optically active amine derivative that can be produced according to the present invention. 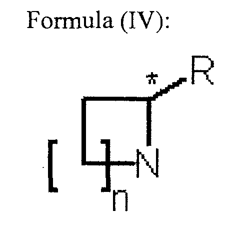 (wherein R group represents an alkyl group having one to three carbon atoms; and n represents an integer of 1 to 4)

In preferred compounds of the present invention, for example, R is a methyl group, ethyl group, or propyl group, and n is 2 or 3 in formula (III) above. More specifically, preferred imine derivatives of the present invention include:
2-methyl-1-pyrroline;
2-methyl-1-piperideine;
2-ethyl-1-pyrroline; and
2-ethyl-1-piperideine.

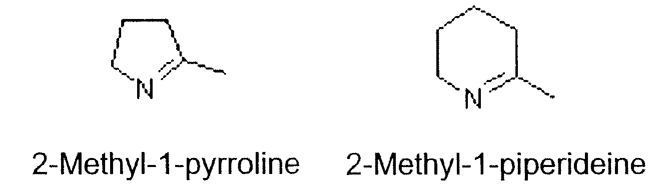

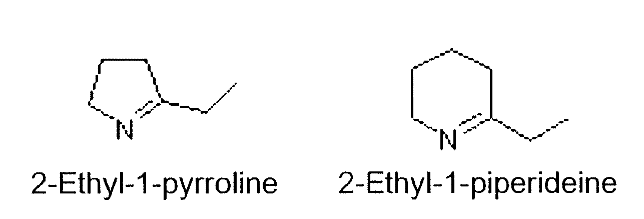

The optically active amine derivatives shown below can be produced by using the above-described compounds as substrates.
2-methyl-1-pyrroline → (S)-2-methylpyrrolidine
2-methyl-1-piperideine → (S)-2-methylpiperidine
2-ethyl-1-pyrroline → (S)-2-ethylpyrrolidine
2-ethyl-1-piperideine → (S)-2-ethylpiperidine

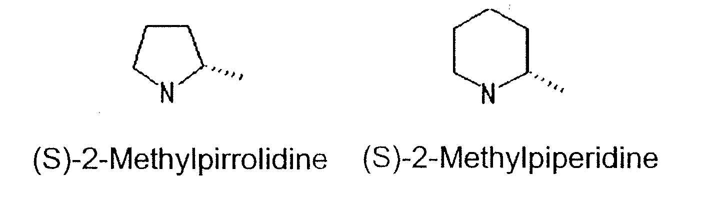

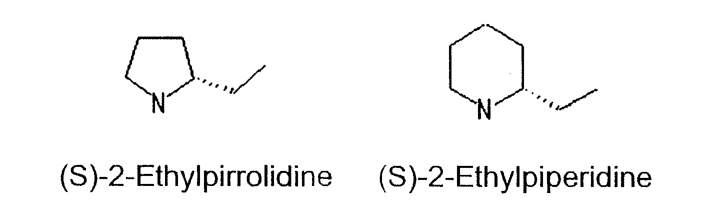

Herein, the ability to stereoselectively reduce an imine derivative refers to the ability to generate an S-isomer amine derivative using as a substrate an imine derivative represented by formula (I) or desirably formula (III) above. The microorganism of the present invention may be any microorganism, as long as it can generate an S-isomer amine derivative. Such microorganisms of the present invention can be obtained by comparing the ability of microorganisms to generate an S-isomer amine derivative for microorganisms belonging to the genus listed below for example.

For example, a test microorganism is cultured in a medium containing an imine derivative represented by compound (I), desirably compound (III), and then the culture medium is assayed to determine the optical purity of the optically active amine derivative generated. As a result, if the generation of an optically active (S)-amine derivative can be demonstrated, the microorganism can be used in the present invention.

Alternatively, a test microorganism is grown in advance in a medium, and the microbial cells are harvested and suspended in an appropriate buffer. Then, the cells are contacted and reacted with an imine derivative represented by compound (I), desirably compound (III). The optical purity of the optically active amine derivative generated in the reaction buffer is determined. When the generation of an optically active amine derivative is demonstrated, the microorganism can be used in the present invention. In this case, when compound (I), preferably compound (III) is added to the culture medium during the culture of the test microorganism, induction of the enzyme for reducing the compound is expected. Furthermore, a more effective accumulation of the product can be expected when an energy source for the reduction is added during the reaction. Such energy sources for the reduction typically include glucose.

The ability of a microorganism to reduce 2-methyl-1-pyrroline can be assessed, for example, by the following procedure. A microorganism is cultured in an appropriate medium containing, if needed, 2-methyl-1-pyrroline. The resulting microbial cells are suspended in a reaction mixture containing 10 mM 2-methyl-1-pyrroline, 25 mM potassium phosphate buffer (pH 7.0), and glucose as needed. The suspension is shaken at 25°C for 24 hours, and then the product is analyzed by TLC or HPLC.

TLC conditions include, for example, a method that uses Silica gel 60 F254 plate, and develops using the developing solvent 1-butanol/acetic acid/water = 2/1/1, followed by color reaction using ninhydrin.

The optical purity of the generated amine derivative can be determined, for example, by the following method.

The generated amine is derivatized with GITC by adding the following components to 50 µl of test sample and incubating the mixture at 40°C for one hour, and is then isolated and quantified by HPLC.
50 µl of 2 mg/ml triethylamine and 100 µl of 8 mg/ml 2,3,4,6-tetra-O-acetyl-β-D-glucopyranosyl isothiocyanate (GITC)

HPLC can be performed, for example, under the following conditions.
- HPLC column: Wakosil II 5C18 (4.6 mm x 150 mm) from Wako Pure Chemical Industries
- elution buffer: 10 mM phosphate buffer (pH 2.5):methanol = 55:45
- flow rate: 1 ml/min
- detection: UV absorbance at 254 nm

Under the conditions described above, the (R)-2-methylpyrrolidine is eluted at 26.3 minutes, while the (S)-2-methylpyrrolidine derivative is eluted at 24.9 minutes.

Needless to say, it is advantageous to use microorganisms with high stereoselectivity to obtain high optical purity products. Specifically, microorganisms which can synthesize optically active S-isomer amine derivatives with an optical purity of, for example 60%, typically 70% or higher, preferably 80% or higher, and more preferably 90% or higher, can be used.

Herein, "optically active amine derivatives" refers to amine derivatives in which a certain type of optical isomer is more abundant than the other optical isomer. In the present invention, preferred optically active amine derivatives have, for example 60%, typically 70% or higher, preferably 80% or higher, and still more preferably 90% or higher optical purity (enantiomeric excess (ee)). The optical purity of optically active amine derivatives can be confirmed with, for example, optical resolution column. In general, the "optical isomer" of the present invention is generally referred to as "optically-active substance" and sometimes also referred to as an "enantiomer".

For example, microorganisms belonging to the genus *Streptomyces* generate S-isomer amine derivatives by reducing imine derivatives represented by formula (I) above.

More specifically, microorganisms belonging to the genus *Streptomyces* can be exemplified by the following microorganism. This microorganism generates high optical purity (S)-amine derivatives and is thus a preferred microorganism of the present invention.

### Streptomyces sp. GF3546.

This microorganism is deposited in the following depositary institution (Date of deposition: June 24, 2008).
(1) Name of depositary institution: Incorporated Administrative Agency National Institute of Technology and Evaluation
(2) Address: Postal code: 292-0818
   2-5-8 Kazusakamatari, Kisarazu-shi, Chiba, Japan
(3) Identification display and accession number:
   *Streptomyces* sp. GF3546
   Accession number: NITE P-593

Furthermore, this microorganism has been transferred to the following international depositary authority based on the Budapest Treaty.
(1) International depositary authority: Incorporated Administrative Agency National Institute of Technology and Evaluation,
   NITE Patent Microorganisms Depositary (NPMD)
(2) Address: Postal code: 292-0818
   2-5-8 Kazusakamatari, Kisarazu-shi, Chiba, Japan
(3) Identification display and accession number:
   *Streptomyces* sp. GF3546
   Accession number: NITE BP-593 (Date of transfer: August 4, 2009)

In the present invention, microorganisms having the ability to stereoselectively reduce an imine derivative represented by formula (I) or (III) described above are not limited to the above deposited bacterial strain. Such microorganisms can be obtained from various microbiological resources. The microbiological resources include those isolated from natural environment and those stored in depositary institutes of microbiology. For example, the microorganisms of the genus *Streptomyces* are preferred microorganisms having the ability to stereoselectively reduce imine derivatives represented by formula (I) or (III) described above. Thus, for example, the above-described selection methods can be used to select microorganisms having an activity of interest from microorganisms of the genus *Streptomyces* deposited in depositary institutes of microbiology. Such microorganisms deposited are available, for example, from the following depositary institutes.
NITE Biological Resource Center (NBRC)
The Institute of Physical and Chemical Research (JCM)
NODAI Culture Collection Center (IAM)
American Type Culture Collection (ATCC)
Deutsche Sammlung von Mikroorganismen (DSM)

Alternatively, microorganisms of the present invention can be selected based on nucleotide sequence information on ribosomal DNA (rDNA). It is well known that the nucleotide sequence information on rDNA is generally useful as an indicator for the genetic distance between organisms. In particular, DNAs encoding 16S rRNA (hereinafter abbreviated as "16S rDNA") have been used as a tool for determining the genetic identity or homology between microorganisms.

16S rRNA is present in all organisms. Although the structure is different between organisms, the sequence features are conserved to a degree that allows sequence alignment. The frequency of horizontal transmission of 16S rRNA between organisms is low, and thus 16S rRNA has been commonly used to classify organisms.

Specifically, small subunit ribosomal RNA (SSU rRNA) is a nucleic acid molecule that constitutes a ribosome which is a site of protein synthesis. The lengths of the nucleotide sequences are slightly different, but the origin is considered to be the same from bacteria to human beings. Thus, the nucleotide sequence of rRNA has been highly conserved during evolution and has been most frequently used in phylogenetic analysis of organisms (Microbiol. Rev., 58, 1-9 (1994)). Prokaryotic SSU rRNA has about 1,500 nucleotides. A systematic taxonomy based on the nucleotide sequence of 16S rRNA is generally used to identify and classify prokaryotes (ASM News, 65, 752-757 (1999); Seibutsu Kogaku Jikkensho (Experimental manual of bioengineering) p. 113, Ed. The Society of Biotechonogy, Japan, Baifukan).

The current classification system of actinomycetes is constituted based on the homology of the nucleotide sequence of 16S rDNA (Stackebrandt, et al. (1997) Int. J. Syst. Bacteriol., 47, 479-491). In the major bacterial systematic taxonomy, bacterial strains are identified not only at the genus level but also at the species level based on the homology of nucleotide sequence of 16S rDNA ("Housenkin no Bunrui to Doutei (Identification and classification of actinomycetes)" (The Society of Actinomycetes Japan) p. 19).

16S rDNA of a bacterial strain identified by the present inventors was found to comprise the nucleotide sequence of SEQ ID NO: 1. Nucleotide sequence information databases can be searched with this nucleotide sequence information to find bacterial strains having a highly homologous nucleotide sequence in their 16S rDNAs from microorganisms whose 16S rDNA has already been determined. Public database service providers, for example, DNA Data Bank of Japan (DDBJ), provide data search services that target the nucleotide sequence information on 16S rRNA (Prokaryotes). Of the microorganisms revealed by this method, microorganisms belonging to the genus *Streptomyces* are selected in particular and can be used in the present invention. If needed, microorganisms selected by the above-described method may be assessed in advance for the activity on imine derivatives.

These microorganisms can be cultured according to methods known in the field of zymology. Culture medium may be a synthetic or natural medium as long as it contains appropriate amounts of carbon source, nitrogen source, inorganic material, and other nutrients. The medium may be a liquid or solid medium.

Specifically, by considering the assimilation in a microorganism to be used, one, two, or more carbon sources are appropriately selected from those commonly used as listed below.
Sugars: glucose, fructose, maltose, galactose;
Natural carbohydrate: starch, starch hydrolysate, molasses, blackstrap molasses, wheat, corn, etc.;
alcohols: glycerol, methanol, ethanol, etc.;
organic acids: acetic acid, gluconic acid, pyruvic acid, ketoglutaric acid, citric acid, etc.;
hydrocarbons: normal paraffin, etc.; and
lipids: palm oil, soybean oil, olive oil, etc.

By considering the assimilation in a microorganism to be used, one, two, or more nitrogen sources are appropriately selected from those commonly used as listed below.

Organic nitrogen compounds: meat extract, peptone, yeast extract, soybean hydrolysate, milk casein, casamino acid, various amino acids, corn steep liquor, other hydrolysates of animals, plants, and microorganisms, etc.

Inorganic nitrogen compounds: ammonia, ammonium nitrate, ammonium sulfate, ammonium salt of sodium chloride or such, nitrate salt of sodium nitrate or such, urea, etc.

Moreover, an inducing substance may be used to enhance the ability of a microorganism to stereoselectively reduce imine derivatives. Imine derivatives can be used as an inducing substance depending on the microorganism used.

Furthermore, it is possible to appropriately select and use as inorganic salts a small amount of one or more phosphoric salt, hydrochloride salt, nitrate salt, acetate salt or the like of magnesium, manganese, potassium, calcium, sodium, copper, zinc, or such. In addition, antifoaming agents such as vegetable oils, detergents, and silicon may be added to the culture medium, if required.

These bacteria can be cultured by conventional culture methods using liquid medium having the above-described components. For example, it is possible to use the following culture methods.
shaking culture
aeration-agitation culture
continuous culture
feeding culture, fed batch culture

Appropriate culture conditions can be selected depending on the type of microorganisms, type of culture, and culture methods. The conditions are not particularly limited as long as they allow the bacterial strain to be used to grow and to have the ability to stereoselectively reduce imine derivatives. General culture conditions are described below.

pH at the start of culture is adjusted to 4 to 10, preferably 6 to 8.

The culture temperature is 15 to 70°C, preferably 20 to 40°C.

The culture period is not particularly limited, as long as it allows expansion of microbial cells having the ability to reduce imine derivatives. Typically, the culture period is 1 to 14 days, preferably 1 to 7 days.

Herein, the "processed materials of microbial cells" refers to materials from treating the microbial cells under conditions that allow them to retain the enzymatic activity of interest. Retainment of enzymatic activity means that the retained enzymatic activity is generally 20% or more as compared to that of viable microbial cells, typically 30% or more, preferably 50% or more, and more preferably 70% or more. The enzymatic activity can be quantitatively compared between viable microbial cells and the processed material thereof by assaying the activity of viable microbial cells and that of the processed material prepared from the same amount of viable microbial cells. In some cases, the enzymatic activity of the processed materials of the present invention may be higher than that of viable microbial cells.

Methods for preparing such processed materials include lyophilization, dehydration drying with an organic solvent, autolysis of microbial cells, extraction of enzymatically active fractions, and sonication. Such processing methods yield, for example, lyophilized microbial cells, acetone-dried microbial cells, microbial cell autolysate, microbial cell extract, crushed product of microbial cells, and sonication product of microbial cells.

These processing methods may be used alone or in combination. For example, after culturing a microorganism in an appropriate culture medium, the microbial cells can be crushed and then centrifuged to prepare cell-free extract. The microbial cells can be crushed mechanically, or by sonication, high pressure treatment, enzymatic digestion, autolysis, or such. Cell-free extracts are included in the processed materials of microbial cells of the present invention.

Furthermore, in the present invention, enzymes that catalyze the reaction can be obtained by partially or completely purifying the microbial cells. More specifically, the present invention relates to imine reductases that can be purified from microbial cells of microorganisms provided by the present invention which have an imine reductase activity. The imine reductases of the present invention have the following physicochemical properties:
(1) activity: to produce (S)-2-methylpyrrolidine by reducing 2-methyl-1-pyrroline in an NADPH-dependent manner and produce 2-methyl-1-pyrroline by oxidizing (S)-2-methylpyrrolidine in an NADP⁺-dependent manner; and
(2) coenzyme dependency: use NADPH as a coenzyme for reduction and use NADP⁺ as a coenzyme for oxidation.

In a preferred embodiment, the imine reductase discovered by the present inventors has, in addition to the properties described above in (1) and (2), the physicochemical properties described below. Such imine reductase can be prepared, for example, from *Streptomyces* sp. GF3546.
(3) optimal pH: pH 7.4 to 8.0 for reduction and pH 10.5 for oxidation.
(4) optimal temperature: 35 to 45°C for reduction.
(5) molecular weight: approximately 30,500 by sodium dodecyl sulfate-polyacrylamide gel electrophoresis (hereinafter abbreviated as SDS-PAGE) and approximately 81,500 by gel filtration.

The imine reductases of the present invention can be purified from the above-described microorganisms, cell-free extract thereof, or such. More specifically, substantially pure imine reductases can be obtained from cell-free extract by using appropriate combinations of the following purification steps:
fractionation based on solubility using ammonium sulfate, acetone, or such;
ion exchange chromatography;
hydrophobic chromatography;
affinity chromatography using 2',5'-ADP Sepharose, blue or red Sepharose, or such; and
gel filtration.

After harvesting microbial cells, an imine reductase of the present invention can be isolated as a substantially pure enzyme, for example, by the following purification steps:
crushing microbial cell by sonication or glass beads, followed by preparation of cell-free extract;
hydrophobic chromatography using Phenylsepharose; and
isolation of the fraction having the imine reductase activity by ion exchange chromatography using MonoQ.

Herein, "substantially pure" means that an enzyme does not substantially contain biological macromolecular compounds or chemical substances other than itself. Such biological macromolecular compounds other than the enzyme include, for example, proteins, sugars, lipids, and nucleic acids, which are derived from microbial cells and culture medium. The purity of a substantially pure enzyme of the present invention is at least 75% or higher, typically 80% or higher, preferably 85% or higher, more preferably 90% or higher, still more preferably 95% or higher, or 99% or higher. Methods for determining enzyme purity are known. Known methods for determining protein purity include, for example, various chromatographic methods and polyacrylamide gel electrophoresis.

The present invention also provides isolated imine reductases having the physicochemical properties described above in (1) and (2). Herein, the isolated imine reductase means that the reductase is separated from the natural environment where it originally occurs. Thus, imine reductases separated from microbial cells are included in the isolated imine reductases of the present invention.

A substantially pure imine reductase of the present invention or isolated imine reductase of the present invention may be formulated into an enzyme composition after purification or isolation. For example, an enzyme composition comprising an imine reductase of the present invention can be prepared by formulating a purified or isolated imine reductase with appropriate carriers. Such carriers include inactive proteins such as albumin, sugars such as sucrose, and sugar alcohols. The enzyme composition may be liquid or in a lyophilized form. Enzyme compositions prepared by lyophilizing aqueous solutions containing the enzyme and carriers are a preferred composition of the present invention.

The activity of an imine reductase of the present invention can be quantitatively determined by the method described below. More specifically, the reductase is incubated at 30°C in a reaction solution (1 mL) having the composition described below. The reaction mixture is assayed to determine the decrease in absorbance at 340 nm due to the decrease of NADPH.
10 mM 2-methyl-1-pyrroline;
100 mM potassium phosphate buffer (pH 7.0);
0.1 mM NADPH; and
enzyme.

The amount of enzyme that catalyzes the decrease of 1 µmol of NADPH per minute under the above-described conditions is defined as 1 U.

The present invention relates to proteins comprising the amino acid sequence of SEQ ID NO: 3. The present invention also includes homologues of a protein comprising the amino acid sequence of SEQ ID NO: 3. A homologue of an imine reductase of the present invention refers to a protein having an amino acid sequence with a deletion, substitution, insertion, and/or addition of one or more amino acids in the amino acid sequence of SEQ ID NO: 3, which is functionally equivalent to a protein comprising the amino acid sequence of SEQ ID NO: 3. Mutations of, for example, 100 amino acid residues or less, typically 50 or less, preferably 30 or less, more preferably 15 or less, and still more preferably 10 or less, or 5 or less are acceptable in the amino acid sequence of SEQ ID NO: 3.

The present invention relates to polynucleotides encoding an imine reductase and homologues thereof. The polynucleotides of the present invention include natural polynucleotides such as DNAs and RNAs, and artificial molecules containing artificial nucleotide derivatives. The polynucleotides of the present invention also include chimeric DNA-RNA molecules. A polynucleotide encoding an imine reductase of the present invention comprises, for example, the nucleotide sequence of SEQ ID NO: 2. The nucleotide sequence of SEQ ID NO: 2 encodes a protein comprising the amino acid sequence of SEQ ID NO: 3. In a preferred embodiment, an imine reductase of the present invention is a protein comprising the amino acid sequence.

A homologue of an imine reductase encoding an imine reductase of the present invention includes an amino acid sequence with a deletion, substitution, insertion, and/or addition of one or more amino acids in the amino acid sequence of SEQ ID NO: 3, and includes a polynucleotide encoding a protein having the physicochemical properties described above in (1) and (2). Those skilled in the art can obtain a polynucleotide homologue by appropriately introducing substitutional, deletional, insertional, and/or additional mutations into the polynucleotide of SEQ ID NO: 2 by site-directed mutagenesis (Nucleic Acid Res. 10, pp. 6487 (1982); Methods in Enzymol. 100, pp. 448 (1983); Molecular Cloning 2nd Edt., Cold Spring Harbor Laboratory Press (1989); PCR A Practical Approach IRL Press pp. 200 (1991)) or the like.

Polynucleotide homologues of the present invention also include polynucleotides that are capable of hybridizing under stringent conditions with a polynucleotide comprising the nucleotide sequence of SEQ ID NO: 2, and which encode a protein having the physicochemical properties (1) and (2) described above. The term "polynucleotides capable of hybridizing under stringent conditions" refers to a polynucleotide that hybridizes to one or more DNA probes that have an arbitrary sequence of at least 20, preferably at least 30, for example, 40, 60, or 100 constitutive nucleotides of SEQ ID NO: 2, using the ECL direct nucleic acid labeling and detection system (GE Healthcare) under conditions described in the manual (washing at 42°C in a primary wash buffer containing 0.5x SSC).

The polynucleotide homologue of the present invention includes a polynucleotide encoding a protein having an identity of at least 70%, preferably at least 80%, much more preferably 90% or higher to the amino acid sequence of SEQ ID NO: 3. Protein identity searches can be performed, for example, against protein amino acid sequence databases such as SWISS-PROT, PIR, and DAD; DNA sequence databases such as DDBJ, EMBL, and GenBank; and databases of amino acid sequences predicted based on DNA sequences by using programs such as BLAST and FASTA *via* the Internet.

The present invention also relates to a protein comprising the amino acid sequence of SEQ ID NO: 3. The present invention also includes homologues of the protein comprising the amino acid sequence of SEQ ID NO: 3. The "imine reductase homologues of the present invention" refers to proteins that are functionally equivalent to a protein comprising the amino acid sequence of SEQ ID NO: 3 and comprise the amino acid sequence with a deletion, substitution, insertion, and/or addition of one or more amino acids in the amino acid sequence of SEQ ID NO: 3. Herein, the phrase "functionally equivalent to the protein comprising the amino acid sequence of SEQ ID NO: 3" means that the protein has the physicochemical properties (1) and (2) described above. Those skilled in the art can obtain a polynucleotide encoding a homologue of the imine reductase by appropriately introducing substitutional, deletional, insertional, and/or additional mutations into the DNA of SEQ ID NO: 2 by site-directed mutagenesis (Nucleic Acid Res. 10, pp. 6487 (1982); Methods in Enzymol. 100, pp. 448 (1983); Molecular Cloning 2nd Edt., Cold Spring Harbor Laboratory Press (1989); PCR A Practical Approach IRL Press pp. 200 (1991)) or the like. A homologue of the imine reductase of SEQ ID NO: 3 can be obtained by introducing and expressing a polynucleotide encoding the imine reductase homologue within a host.

Furthermore, the imine reductase homologue of the present invention refers to a protein having an identity of at least 70%, preferably at least 80%, more preferably 90%, much more preferably 90% or higher to the amino acid sequence of SEQ ID NO: 3. Protein identity searches can be performed against protein amino acid sequence databases, such as SWISS-PROT, PIR, and DAD; DNA sequence databases, such as DDBJ, EMBL, and GenBank; and databases of amino acid sequences predicted based on DNA sequences by using programs such as BLAST and FASTA *via* the Internet.

Polynucleotides encoding the imine reductases of the present invention can be isolated, for example, by the methods described below.

PCR can be performed to obtain the DNAs of the present invention, using PCR primers designed based on the nucleotide sequence of SEQ ID NO: 2, and either chromosomal DNA from a strain producing the enzyme, or a cDNA library, as a template.

Additionally, the polynucleotides of the present invention can be obtained by digesting chromosomal DNA from a strain producing the enzyme with restriction enzymes, and then inserting these fragments into a phage, plasmid, or the like; and transforming *E. coli* with the DNA construct. The resulting library, or a cDNA library, can be screened by colony hybridization, plaque hybridization, or similar methods, using the DNA fragment obtained as described above as a probe.

Alternatively, the polynucleotides of the present invention can be obtained by analyzing the nucleotide sequence of the DNA fragment yielded by PCR, and then designing PCR primers for sequence extension based on the sequence obtained. Next, chromosomal DNA of the enzyme-producing strain is digested with appropriate restriction enzymes, and then subjected to self-circle formation to serve as a template for inverse PCR (Genetics 120, 621-623 (1988)), or alternatively by RACE (Rapid Amplification of cDNA End; "Experimental manual for PCR" p.25-33, HBJ Press), and such.

The polynucleotides of the present invention include synthetic DNAs in addition to genomic DNAs and cDNAs cloned by the method described above.

The present invention also provides expression vectors for the imine reductase which are constructed by inserting a polynucleotide encoding the imine reductase of the present invention isolated by the method described above into a conventional expression vector.

The imine reductase of the present invention can be obtained from recombinants by culturing the recombinants transformed with the expression vector.

There is no limitation on the type of microorganism to be transformed for the expression of the imine reductase of the present invention, as long as it is capable of being transformed with a recombinant vector that contains a polynucleotide encoding a polypeptide encoding the imine reductase and is competent to express imine reductase activity. The available microorganisms include, the microorganisms listed below:
bacterial strains for which host-vector systems are available, such as those belonging to:
genus *Escherichia*,
genus *Bacillus*,
genus *Pseudomonas*,
genus *Serratia*,
genus *Brevibacterium*,
genus *Corynebacterium*,
genus *Streptococcus*, and
genus *Lactobacillus*;
actinomycetes for which host-vector systems are available, such as those belonging to:
genus *Rhodococcus*, and
genus *Streptomyces*;
yeasts for which host-vector systems are available, such as those belonging to:
genus *Saccharomyces*,
genus *Kluyveromyces*,
genus *Schizosaccharomyces*,
genus *Zygosaccharomyces*,
genus *Yarrowia*,
genus *Trichosporon*,
genus *Rhodosporidium*,
genus *Pichia*, and
genus *Candida*; and
molds for which host-vector systems are available, such as those belonging to:
genus *Neurospora*,
genus *Aspergillus*,
genus *Cephalosporium*, and
genus *Trichoderma*.

Preparation of transformants and construction of recombinant vectors compatible to each host can be achieved by using conventional techniques of molecular biology, bioengineering, and genetic engineering (for example, Sambrook et al., Molecular Cloning, Cold Spring Harbor Laboratories). To obtain expression of the genes of the present invention for imine reductases that use NADPH as an electron donor in a microorganism, the DNA should be first inserted into a plasmid or phage vector that is stable in the microorganism and which will permit transcription and translation of the genetic information.

To achieve this, a promoter, which is a regulatory unit for transcription and translation, is inserted at the 5'-side (upstream) of the DNA chain of the present invention, and more preferably a terminator is also inserted at the 3'-side (downstream) of the DNA. Such promoters and terminators should be known to be functional in the host microorganisms. Such vectors, promoters, terminators, and others, which can be used with various microorganisms, are described in detail in "Fundamental Microbiology (Biseibutsugaku Kiso-kouza) 8: Genetic Engineering, KYORITSU SHUPPAN CO., LTD.", and in particular those to be used with yeasts are described in detail in: Adv. Biochem. Eng. 43, 75-102 (1990); Yeast 8, 423-488 (1992); and others.

For example, for bacterial species belonging to the genus *Escherichia*, in particular, *Escherichia coli*, pBR and pUC plasmids are available as plasmid vectors, and available promoters include those derived from lac (β-galactosidase), trp (tryptophan operon), tac, trc (fusion of lac and trp), and λ phage PL and PR. Available terminators include those derived from trpA, phage, and rrnB ribosomal RNA. Suitable vectors include pSE420D (described in Japanese Patent Application Kokai Publication No. (JP-A) 2000-189170 (unexamined, published Japanese patent application)), which has a modified cloning site derived from the multi-cloning site of the commercially available pSE420 (Invitrogen).

For bacterial species belonging to the genus *Bacillus,* available vectors include pUB 110 plasmids and pC194 plasmids. The plasmids can be integrated into the chromosome. Promoters and terminators are available from genes including apr (alkaline protease), npr (neutral protease), amy (α-amylase).

For *Pseudomonas putida*, *Pseudomonas cepacia*, and other species from the genus *Pseudomonas*, host-vector systems have been developed previously. Wide host-range vectors (containing genes required for autonomous replication derived from RSF1010 and others), such as pKT240, are available; and these vectors were constructed from the plasmid TOL which is involved in the degradation of toluene and toluene-containing compounds. The lipase gene (JP-A (Kokai) H05-284973) can provide the promoter and terminator.

For bacterial species belonging to the genus *Brevibacterium*, in particular, *Brevibacterium lactofermentum*, available plasmid vectors include pAJ43 (Gene 39, 281 (1985)). This species can utilize promoters and terminators that are suitable for *E. coli,* without modification.

For bacterial species belonging to the genus *Corynebacterium*, in particular, *Corynebacterium glutamicum*, plasmid vectors such as pCS11 (JP-A (Kokai) S57-183799) and pCB101 (Mol. Gen. Genet. 196, 175 (1984)) are available.

For bacterial species belonging to the genus *Streptococcus*, plasmid vectors such as pHV1301 (FEMS Microbiol. Lett. 26, 239 (1985)) and pGK1 (Appl. Environ. Microbiol. 50, 94 (1985)) are available.

For bacterial species belonging to the genus *Lactobacillus*, the plasmid vector pAM β1 (J. Bacteriol. 137, 614 (1979)) is available; and this plasmid vector was constructed for bacterial species belonging to the genus *Streptococcus*, and promoters which are suitable for *E. coli* can also be used.

For bacterial species belonging to the genus *Rhodococcus*, plasmid vectors isolated from *Rhodococcus rhodochrous* (J. Gen. Microbiol. 138, 1003 (1992)) are available.

For bacterial species belonging to the genus *Streptomyces,* plasmids can be constructed by the method of Hopwood et al. which is described in Genetic Manipulation of Streptomyces: A Laboratory Manual Cold Spring Harbor Laboratories (1985). In particular for *Streptomyces lividans*, available plasmid vectors are pIJ486 (Mol. Gen. Genet. 203, 468-478 (1986)), pKC1064 (Gene 103, 97-99 (1991)), and pUWL-KS (Gene 165, 149-150 (1995)). Furthermore, the same plasmids can be used for *Streptomyces virginiae* (Actinomycetol. 11, 46-53 (1997)).

For fungal species belonging to the genus *Saccharomyces*, in particular, *Saccharomyces cerevisiae*, YRp plasmids, YEp plasmids, YCp plasmids, and YIp plasmids are available. Integration vectors (*e.g.*, EP 537456) which use homologous recombination with multicopy ribosomal DNA on the chromosome are highly advantageous since a gene of interest can be introduced in multiple copies using the vector, and the integrated gene is stable in the host. Available promoters and terminators are derived from alcohol dehydrogenase (ADH), glyceraldehyde-3-phosphate dehydrogenase (GAPDH), acid phosphatase (PHO), β-galactosidase (GAL), phosphoglycerate kinase (PGK), enolase (ENO), and others.

For fungal species belonging to the genus *Kluyveromyces,* in particular, *Kluyveromyces lactis*, available plasmid vectors include *Saccharomyces cerevisiae*-derived 2µm plasmids and pKD1 plasmids (J. Bacteriol. 145, 382-390 (1981)), plasmids derived from pGK11 involved in killer activity, KARS plasmids derived from genes associated with the autonomous replication in fungal species belonging to the genus *Kluyveromyces,* and vector plasmids capable of integrating into the chromosome *via* homologous recombination with ribosomal DNA (*e.g.*, EP 537456). Promoters and terminators derived from ADH, PGK, and other genes are also available.

For fungal species belonging to the genus *Schizosaccharomyces*, available plasmid vectors are: ARS derived from *Schizosaccharomyces pombe* (genes involved in the autonomous replication) and *Saccharomyces cerevisiae*-derived plasmid vectors containing selection markers which complement nutritional requirement (Mol. Cell. Biol. 6, 80 (1986)). In addition, the ADH promoter and others derived from *Schizosaccharomyces pombe* are available (EMBO J. 6, 729 (1987)). In particular, the commercially available pAUR224 (Takara Bio Inc.) can be readily used.

For fungal species belonging to the genus *Zygosaccharomyces*, plasmid vectors derived from the *Zygosaccharomyces* rouxii-derived plasmid pSB3 (Nucleic Acids Res. 13, 4267 (1985)) or the like are available, and available promoters include the PHO5 promoter derived from *Saccharomyces cerevisiae* and the GAP-Zr (glyceraldehyde-3-phosphate dehydrogenase) promoter derived from *Zygosaccharomyces rouxii* (Agri. Biol. Chem. 54, 2521 (1990)).

For *Pichia angusta* (previous name: *Hansenula polymorpha*) belonging to the genus *Pichia*, there are previously developed host-vector systems. *Pichia angusta*-derived genes involved in the autonomous replication (HARS1 and HARS2) can be used as vectors. However, these genes are relatively unstable, and therefore, multicopy integration into the chromosome (Yeast 7, 431-443 (1991)) may be used more advantageously. The AOX (alcohol oxidase) promoter which is inducible with methanol or the like, or the FDH (formate dehydrogenase) promoter can be used for this fungal species. In addition, for *Pichia pastoris* and others, host-vector systems using *Pichia*-derived genes involved in the autonomous replication (PARS 1 and PARS2) or the like have been developed (Mol. Cell. Biol. 5, 3376 (1985)). Thus, it is possible to use high efficiency promoters such as AOX which is inducible with high density culture and methanol (Nucleic Acids Res. 15, 3859 (1987)).

For *Candida maltosa*, *Candida albicans*, *Candida tropicalis*, *Candida utilis*, and other species belonging to the genus *Candida*, host-vector systems have been developed. *Candida maltosa*-derived ARS has been cloned previously (Agri. Biol. Chem. 51, 51, 1587 (1987)), and thus vectors using this ARS have been developed for *Candida maltosa*. In addition, chromosome integration vectors with high efficiency promoters have been developed for *Candida utilis* (JP-A (Kokai) H08-173170).

The fungal species *Aspergillus niger* and *Aspergillus oryzae*, which belong to the genus *Aspergillus*, have been extensively studied. Plasmids, chromosome integration vectors, and extracellular protease and amylase derived promoters are also available for the fungal species (Trends in Biotechnology 7, 283-287 (1989)).

For *Trichoderma reesei*, belonging to the genus *Trichoderma*, a host-vector system has been developed, and an extracellular cellulase gene-derived promoter and others are available (Biotechnology 7, 596-603 (1989)).

Furthermore, various host-vector systems have been developed for plant and animal species, in addition to microorganisms. Large-scale systems for the expression of foreign proteins in insects, in particularly, silkworms (Nature 315, 592-594 (1985)), and plants such as colza, maize, and potato have been developed and are suitable for use.

Microorganisms having the ability to produce imine reductases of the present invention include all bacterial strains that belong to the genus *Streptomyces* and have the ability to produce imine reductases, mutants and variants thereof, and transformed strains which gain the ability to produce the enzyme of the present invention prepared by genetic engineering techniques.

Methods for producing optically active amine derivatives of the present invention comprise a step of contacting a substrate compound represented by formula (I) or (III) with enzymatically active materials having an imine reductase activity, which is the ability to stereoselectively reduce the substrate compound under conditions that enable the reduction of the substrate compound by the enzymatically active materials. Herein, the "enzymatically active materials" refers to materials that are derived from a microorganism having the ability to stereoselectively reduce the substrate compound represented by formula (I) or (III) and retain the ability. Specifically, for example, the components described below are included in the enzymatically active materials of the present invention, as long as they retain the imine reductase activity.
(i) Microorganisms having the ability to stereoselectively reduce the substrate compound represented by formula (I) or (III);
(ii) culture of cells of the microorganisms described in (i); and
(iii) processed products of cells of the microorganisms described in (i).

The microorganisms of the present invention may be culture or microbial cells. The culture refers to a condition in which the microorganisms exist with the medium that supports the survival and growth of the microorganisms. For example, liquid medium containing microorganisms is the culture. Meanwhile, as used herein, "microbial cell" is a term that refers to a microorganism itself. Microbial cells are cells of a microorganism collected from the culture. For example, microbial cells of a microorganism can be collected from liquid medium by centrifugation. The components of medium may be removed from the surface of microbial cells by washing the microbial cells collected from the culture.

Without any modification or after immobilization, the enzymatically active materials of the present invention may be contacted with substrate compounds. There are various known methods for immobilizing microbial cells or processed products thereof. Such known methods for immobilizing microbial cells or processed products thereof include, for example:
polyacrylamide method;
sulfur-containing polysaccharide method (κ-carrageenan gel method, etc.);
alginic acid gel method;
agar gel method; and
ion exchange resin method.

Alternatively, microorganisms or processed products thereof may be reacted by contacting them with substrate compounds in a membrane bioreactor using ultrafiltration membrane or the like.

The stereoselective imine reduction by the microorganisms of the present invention can be carried out by the following procedure. The microorganisms are cultured under the above-described culture conditions suitable for inducing the enzyme; an imine derivative is added as a reaction substrate to the prepared culture, microbial cells collected from the culture, or processed product thereof; and the mixture is incubated. Alternatively, an imine derivative which serves as a reaction substrate during culture is added at the start of culture of microorganism described above. Thus, the stereoselective imine reduction occurs during culture.

With microorganisms of the present invention, stereoselective reduction can be achieved by the following procedure. The microorganisms are cultured under the above-described culture conditions suitable for inducing the enzyme; and a reaction substrate is added to the prepared culture, microbial cells collected from the culture, or a processed product thereof. Alternatively, the reduction may be conducted at the same pH and temperature ranges as the above-described culture conditions, simultaneously with culturing for one to seven days.

Such reaction conditions include, for example, those shown below:
pH: 4.0 to 9.0, preferably 6.0 to 8.0;
temperature: 15 to 50°C, preferably 20 to 40°C; and
reaction time: contacting for four hours to seven days.

In general, contamination of impurity in the medium of the reaction system can be minimized by performing the culture of the microorganisms and reaction individually. This can be advantageous when the product is purified after reaction.

Furthermore, a more efficient enzymatic reduction reaction can be expected by performing the reaction in the presence of sugars or such as a reduction energy source. Compounds that are added as reduction energy sources can be added according to the amount of imine derivative as a reaction substrate. More specifically, the following can be used as reduction energy sources.
sugars: glucose, glucose-6-phosphate, sucrose, etc.
organic acids: malic acid, citric acid, formic acid, etc.
amino acids: alanine, glutamic acid, lysine, etc.
alcohols: ethanol, isopropanol, etc.

In addition, inorganic compounds such as phosphorous acid can be used as reduction energy sources in some cases.

The reduction reaction of the present invention proceeds in the presence of a hydrogen donor. Accordingly, the condition that enables the reduction of a substrate compound in the present invention is achieved by incubating the substrate compound with enzymatically active materials in the presence of a hydrogen donor. Any hydrogen donor can be used as long as it supports the reduction reaction of the present invention. Preferred hydrogen donors of the present invention include NADPH and NADH. NADPH is preferably used in combination with an imine reductase derived from *Streptomyces* sp. GF3546, which was successfully isolated by the present inventors, homologues thereof, or an enzymatically active material thereof.

The regeneration of NADPH from NADP⁺, which is generated from NADPH accompanying the reduction reaction described above, can be achieved using the NADP⁺-reducing ability of a microorganism (glycolytic system, the assimilation pathway of methylotroph C1 compound, etc.). The NADP⁺-reducing ability can be enhanced by adding glucose, ethanol, or the like to the reaction system. Alternatively, the regeneration can be achieved by adding to the reaction system a microorganism that has the ability to generate NADPH from NADP⁺, processed product thereof, or enzyme thereof. For example, NADPH can be regenerated by using microorganisms having glucose dehydrogenase, alcohol dehydrogenase, amino acid dehydrogenase, organic acid dehydrogenase (malate dehydrogenase, etc.), or such, processed products thereof, or purified or partially purified enzymes thereof. These components which constitute the reaction required for the NADPH regeneration can be added to the reaction system for producing optically active amines of the present invention, added after immobilization, or contacted through an NADPH-exchangeable membrane.

Alternatively, the additional reaction system for NADPH regeneration may be unnecessary in some cases when viable microbial cells of a microorganism transformed with a recombinant vector carrying a DNA of the present invention are used in the above-described methods for producing optically active amines. More specifically, efficient reduction can be achieved without adding NADPH regenerating enzyme by using transformed microorganisms having a strong activity of regenerating NADPH. Furthermore, more efficient imine reduction reaction can be achieved by co-expressing an imine reductase with an NADPH regenerating enzyme as follows. A DNA encoding an imine reductase of the present invention is introduced into a host, along with a gene available for NADPH regeneration, such as a gene encoding glucose dehydrogenase, alcohol dehydrogenase, amino acid dehydrogenase, or organic acid dehydrogenase (malate dehydrogenase, etc.). To avoid incompatibility when introducing two or more of such genes into hosts, the respective genes are individually inserted into different recombinant vectors whose replication origins are different from one another, and hosts are transformed with these vectors. Alternatively, two genes are inserted into a single vector, and both or one of the genes are integrated into the chromosome.

When multiple genes are inserted into a single vector, regulatory regions for expression such as the promoter and terminator may be linked to each gene. Alternatively, the genes may be expressed as an operon containing multiple cistrons, such as the lactose operon.

For example, glucose dehydrogenases derived from the species that belongs to *Bacillus subtilis* or *Thermoplasma acidophilum* can be used as an NADPH regenerating enzymes. Specifically, preferred recombinant vectors include pSG-S2MP, which contains the genes encoding the imine reductase and the glucose dehydrogenase derived from *Bacillus subtilis* as inserts.

When there are changes in the pH of a reaction solution in association with the degradation of amine derivatives, the reaction can be continued by regulating pH within a certain range using an appropriate acid or base and by keeping good reactivity.

When viable microbial cells are used in the present invention, in some cases the reaction period can be shortened by adding a detergent to the reaction mixture. Detergents to be used for this purpose are not particularly limited, as long as they increase the permeability of the cell wall of viable microbial cells. Such detergents include cetylpyridinium bromide, cetyltrimethylammonium bromide, Triton X-100, paraisooctylphenyl ether, Tween80, and Span 60. The detergents are preferably used at about 0.0001 to 1% in the reaction mixture.

Alternatively, the same effect can be expected by adding an organic solvent to the reaction solution in some cases. Detergents to be used for this purpose are not particularly limited, as long as they increase the permeability of the cell wall of viable microbial cells. Such organic solvents include toluene and xylene. The organic solvents are preferably used at about 0.0001 to 1% in the reaction solution.

Alternatively, instead of making additions to the reaction solution, it is possible to use microbial cells pre-treated with water or buffer containing a detergent or organic solvent after cell harvest to increase the permeability of the cell wall.

Imine derivatives which are used as a reaction substrate of the present invention can be used at a concentration adequate for efficient production of the target product. In some cases, imine derivatives are toxic to microbial cells or the enzyme that catalyzes the reaction. Thus, it is noteworthy that reactions with a high concentration of the imine derivatives may not necessarily result in efficient production. The concentration of imine derivatives in the reaction mixture ranges from, for example, 0.05 to 50%w/v, preferably 0.1 to 10%w/v. Imine derivatives can be added by any method such as batch method, fed-batch method, and feed method. Alternatively, instead of adding the substrate imine, a ketone and an amine, which are raw materials for imine, are added to generate an imine in the reaction solution. The generated imine can serve as substrate.

Product quality can be improved by treating imine derivatives to be added under an inactive gas atmosphere to prevent coloration. With such expected effect, raw materials can also be added to the reaction mixture under inactive gas atmosphere. Furthermore, coloration can be prevented by performing the enzyme reaction under an inactive gas atmosphere. Such inactive gas used herein includes, for example, nitrogen, argon, and helium. In particular, nitrogen is an inactive gas that is easy to obtain. Alternatively, argon does not readily diffuse out because its specific gravity is higher than that of air. Thus, argon is operationally advantageous in that the inactive gas atmosphere can be maintained even in an open operation.

The present invention includes the step of collecting optically active amine derivatives obtained by contacting substrate compounds with an enzymatically active material.
Specifically, optically active amine derivatives obtained *via* stereoselective reduction of imine derivatives can be isolated from the reaction solution by the combined use of various chromatographies, crystallization, and the like. Optically active amine derivatives accumulated in the reaction solution can be extracted, for example, by using the following solvents:
ethyl acetate;
butyl acetate;
toluene;
xylene;
hexane;
heptane;
methylisobutylketone;
methyl-t-butyl ether; and
organic solvents such as halogen series.

The extracted amine derivatives can be further purified by the chromatographies described below. Before purification, treatments such as washing with acid or alkali, and dehydration with a dehydrating agent can be used in combination if needed.
ion exchange chromatography
adsorption chromatography

For example, after insoluble materials such as microbial cells are removed from the reaction mixture by centrifugation, extraction is performed using a solvent under an alkaline condition. Optically active amine derivatives can be collected by concentrating the extract under reduced pressure.

Insoluble materials such as microbial cells are removed from the solution, and the solution is adsorbed onto ion exchange resins or the like which can adsorb amine derivatives. A high concentration solution can be easily obtained by eluting the amine derivatives with alkali such as ammonia water, aqueous solution of sodium hydroxide, methanol solution of sodium hydroxide, or ethanol solution of sodium hydroxide.

Alternatively, insoluble materials such as microbial cells are removed from the solution, and the solution is adsorbed onto ion exchange resins or the like which can adsorb amine derivatives. A high concentration solution can be easily obtained by eluting the amine derivatives with alkali such as ammonia water, aqueous solution of sodium hydroxide, methanol solution of sodium hydroxide, or ethanol solution of sodium hydroxide.

Alternatively, after solvent extraction, the extract is converted into hydrochloride salt by treating it with hydrochloric acid to collect the optically active amine derivative as crystal. To improve the yield, the solvent may be concentrated under reduced pressure or replaced with a solvent suitable for crystallization.

Furthermore, to improve the yield, the solvent may be concentrated under reduced pressure or replaced with a solvent suitable for crystallization.

Alternatively, in some cases the optical purity of optically active amine derivatives can be increased by preparing them as crystallized salts with acid. Optically active amine derivatives can be collected as a crystal by chloridizing with hydrochloric acid after solvent extraction. Such acids for purifying the salts include mineral acids such as hydrochloric acid and sulfuric acid, and organic acids such as mandelic acid, citric acid, and tartaric acid. The salts of amine derivatives obtained by the methods described above are dissolved in strong acid or strong alkali, and then neutralized with an appropriate alkali or acid to achieve neutralization crystallization. This can readily separate the amine derivatives from other impurities. For example, mineral acids such as hydrochloric acid and sulfuric acid can be used as strongly acidic solution to dissolve the salts of amine derivatives. Meanwhile, aqueous solutions of NaOH and ammonia water can be used as strong alkaline solution.

In addition, amine derivatives can be easily separated from other impurities by heating the aqueous solution, and then cooling to re-crystallize.

The amine derivatives can be further purified to high purity by conducting silica gel chromatography using an appropriate developing solvent after dissolving the obtained crystals. Such developing solvents for the silica gel chromatography include, for example, butanol, acetic acid, mixed solvents thereof with water.

All prior art documents cited in the specification are incorporated herein by reference.

### Examples

Hereinbelow, the present invention will be specifically described with reference to the Examples, but it is not to be construed as being limited thereto.

In the tables, "GF" in the bacterial strain names indicates that the strains are held by Gifu University, National University Corporation.

### [Example 1] Screening

Liquid culture medium (pH was adjusted at 7.3) containing 4 g/l yeast extract, 10 g/l malt extract, 4 g/l glucose, 5 mg/l ferric sulfate heptahydrate, and 1 g/l 2-methyl-1-pyrroline was prepared and aliquoted (4 ml) into 18 mm diameter test tubes. The tubes were sterilized by heating at 121°C for 15 minutes in an autoclave. Bacterial strains shown in Table 1 below were each inoculated to the tubes with a platinum loop. The tubes were incubated at 28°C while shaking for two to six days.

The resulting culture media were centrifuged to collect the microbial cells. 25 mM phosphate buffer (pH 7.0) containing 10 mM 2-methyl-1-pyrroline was added to the cells. The cells were reacted at 25°C while shaking for 24 hours. After the reaction, the cells were removed by centrifugation. Then, TLC analysis was carried out using silica gel (developing solvent: 1-butanol/acetic acid/water = 2/1/1), followed by detection using ninhydrin spray.

As for the cells under reaction, 50 µl of 2 mg/ml triethylamine aqueous solution and 100 µl of 8 mg/ml 2,3,4,6-tetra-O-acetylglucopyranosyl isothiocyanate (GITC) were added to 50 µl of the supernatant, and the mixture was incubated at 40°C for one hour for derivatization with GITC. Optical purity was measured by liquid chromatography analysis. C 18 column from Wako Pure Chemical Industries (Wakosil II 5C18, 4.6 mm x 150 mm) was used, and an elution buffer of 10 mM phosphate buffer (pH 2.5)/methanol = 55/45 was loaded at the flow rate of 1ml/min. Optical purity was measured by detecting UV absorbance at 254 nm.

The analysis result is shown in Table 1. The result demonstrated production of optically active 2-methylpyrrolidine.

**[Table 1]**

| Strain name | Cumulative dose (mM) | Optical purity (%ee) |
|---|---|---|
| Streptomyces sp. GF 3546 | 0. 8 4 m M | 8 1 . 3 (S) |

### [Example 2] Identification of GF3 546

The microbiological features of GF3546 are summarized in Table 2.

**[Table 2]**

| **SII05767-01** | |
|---|---|
| 1. MACROSCOPIC OBSERVATION | |
| GROWING CONDITIONS FOR EACH MEDIUM | CULTURE AT 30°C FOR 3 DAYS OR MORE |
| **ISP2** | |
| COLONY SIZE | φ **2.0-3.0 mm** |
| COLONY SURFACE SHAPE | COTTON SHAPED |
| COLONY COLOR: | |
| SURFACE (AERIAL HYPHA) | GRAY |
| REAR SURFACE (BASAL HYPHA) | YELLOW |
| WATER-SOLUBLE PIGMENT PRODUCITON | BROWN WATER-SOLUBLE PIGMENT PRODUCED |
| **ISP3** | SURFACE : GRAY REAR SURFACE: BROWN |
| **ISP4** | SURFACE : WHITE REAR SURFACE: PALE YELLOW |
| **ISP5** | SURFACE : PALE YELLOW REAR SURFACE : PALE YELLOW |

| 2. MICROSCOPIC OBSERVATION | |
|---|---|
| SHAPE OF AERIAL HYPHA | BRANCHED |
| | WIDTH 0.9-1.0mm |
| | HELICAL AERIAL MYCELIUM OBSERVED |
| 3. PHYSIOLOGICAL AND BIOCHEMICAL | PROPERTIES |
| GELATIN LIQUEFACTION | + |
| STARCH HYDROLYSIS | + |
| NITRATE-REDUCING REACTION | - |
| PEPTONZATION/COAGULATION OF SKIM MILK | - |
| RANGE OF GROWING TEMPERATURE (°C) | |
| **20** | + |
| **25** | + |
| **30** | + |
| **37** | + |
| **45** | - |
| SALT TOLERANCE (%) | |
| **4.0** | - |
| **7.0** | - |
| **10.0** | - |
| **13.0** | - |
| CARBON SOURCE AVAILABILITY | |
| ISP MEDIUM No. 9 | - |
| GLUCOSE | + |
| L-RHAMNOSE | + w |
| D-MANNITOL | + |
| D-FRUCTOSE | + |
| L-ARABINOSE | + w |
| RAFFINOSE | - |
| SUCROSE | - |
| D-XYLOSE | + |
| INOSITOL | - |
| MELANIN-LIKE PIGMENT PRODUCTION | |
| ISP MEDIUM No. 6 | - |
| ISP MEDIUM No. 7 | - |

| | |
|---|---|
| + : POSITIVE - : NEGATIVE w : WEAK RESPONSE | |

Then, GF3546 was identified by 16S rDNA analysis. The nucleotide sequence of 16S rDNA was determined using the MicroSeq Full Gene 16S rDNA PCR/Sequencing kit (ABI). The analysis was performed according to the procedure described in the instruction manual of the kit. The determined nucleotide sequence is shown in SEQ ID NO: 1. The nucleotide sequence of SEQ ID NO: 1 was searched against APOLLON DB (product name; TechnoSuruga Laboratory) by BLAST. The result showed that the sequence exhibited 98.6% identity to the 16S rDNA sequences of the following bacterial strains:
*Streptomyces pulveraceus* NBRC3855;
*Streptomyces gelaticus* NBRC12866; and
*Streptomyces sannanensis* NBRC14239.

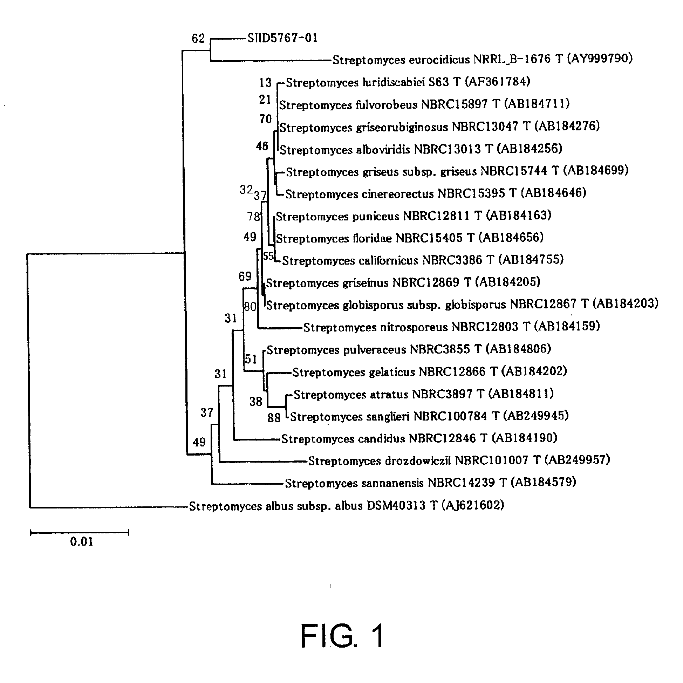

A molecular phylogenetic tree was constructed based on the result and is shown in Fig. 1. The result of cluster analysis suggested that GF3546 belongs to *Streptomyces* sp.

### [Example 3] Synthesis of (S)-2-methylpyrrolidine by GF3546

Liquid culture medium (pH was adjusted at 7.3) containing 20 g/l yeast extract, 30 g/l meat extract, 10 g/l NZ amine, 20 g/l glucose, 1 mg/l ferric sulfate heptahydrate, 1 mg/l manganese chloride tetrahydrate, and 1 mg/l zinc sulfate heptahydrate was aliquoted (40 ml) into shaking flasks. The flasks were sterilized by heating at 121°C for 15 minutes in an autoclave. *Streptomyces* sp. GF3546 was inoculated to the flasks with a platinum loop. The flasks were incubated at 28°C while shaking for 72 hours.

The resulting culture media were filtrated to collect the microbial cells. 30 mM 2-methyl-1-pyrroline and 100 mM phosphate buffer containing 2% glucose (pH 7.0) were added to the cells. The cells were reacted at 25°C while shaking.

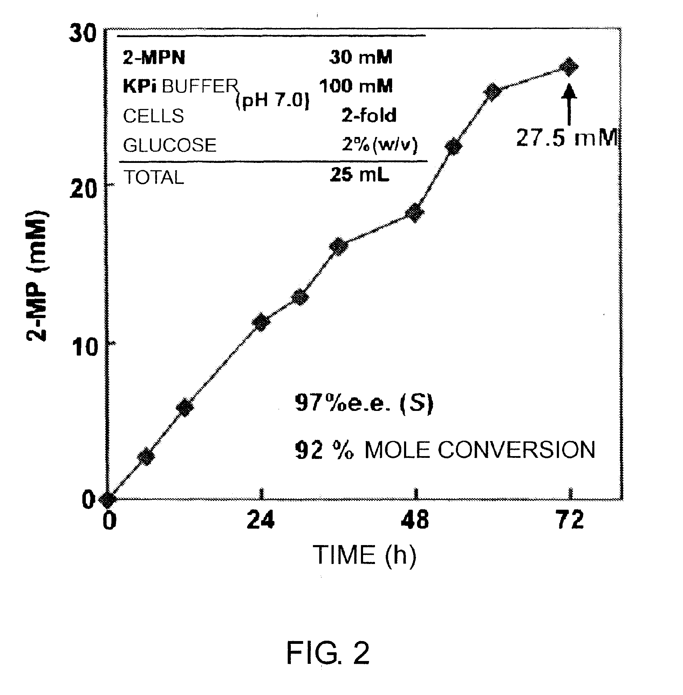

After reaction, derivatization with GITC was carried out appropriately according to Example 1. Then, the product was analyzed by HPLC. The result is shown in Fig. 2. The result showed that (S)-2-methylpyrrolidine was generated up to 27.5 mM with 97%ee selectivity after 72 hours of reaction. The optical purity was determined by the method described below.

The ingredients described below were added to 50 µl of reaction solution, and the resulting mixture was incubated at 40°C for one hour. The produced amine was derivatized with GITC, and then isolated by HPLC and quantified. 50 µl of 2 mg/ml triethylamine; and
100 µl of 8 mg/ml 2,3,4,6-tetra-O-acetyl-β-D-glucopyranosyl isothiocyanate (GITC).

The conditions of HPLC were as follows:
- HPLC column: Wakosil II 5C18 (4.6 mm x 150 mm) from Wako Pure Chemical Industries
- elution buffer: 10 mM phosphate buffer (pH 2.5)/methanol = 55/45
- flow rate: 1 ml/min
- detection: UV absorbance at 254 nm

Under the conditions described above, (R)-2-methylpyrrolidine was eluted at 26.3 minutes, while (S)-2-methylpyrrolidine was eluted at 24.9 minutes. The optical purity of each eluted fraction was determined based on the UV absorbance at 254 nm.

### [Example 4] Purification of imine reductase from GF3546

Strain GF3546 was cultured by the method described in Example 3, and the microbial cells were harvested by filtration. The resulting wet microbial cells were suspended in 100 mM potassium phosphate buffer (pH 7.0) containing 1 mM dithiothreitol (DTT) and 2 mM phenylmethanesulfonyl fluoride (PMSF), and crushed in Bead Beater (Biospec). Then, the cell debris were removed by centrifugation to give a cell-free extract. Protamine sulfate was added to the cell-free extract, and centrifuged to give nucleic acid-free supernatant. Ammonium sulfate was added to the supernatant until the saturation degree reached 30%. The resulting mixture was loaded onto PhenylSepharose HP 26/10 equilibrated with standard buffer (10 mM potassium phosphate buffer (pH 7.0) containing 1mM DTT and 5% glycerol) 30%-saturated with ammonium sulfate. The PhenylSepharose HP 26/10 was eluted with an ammonium sulfate concentration gradient of 30% to 0% saturation. The imine-reducing activity was detected in some of the gradient-eluted fractions. The eluted peak fractions were collected and concentrated by ultrafiltration. The imine-reducing activity was determined by the assay method described below.

### Standard method for assaying imine-reducing activity

A reaction mixture containing the enzyme, 100 mM potassium phosphate buffer (pH 7.0), 0.2 mM NADPH, and 10 mM 2-methyl-1-pyrrolin was incubated at 30°C. The reaction mixture was assayed to determine the decrease in absorbance at 340 nm due to the decrease of NADPH. The amount of enzyme that catalyzes the decrease of 1 µmol of NADPH per minute was defined as 1 U.

The concentrated enzyme solution was dialyzed against standard buffer, and then loaded onto MonoQ 16/10 equilibrated with the same buffer. MonoQ 16/10 was eluted with a gradient of sodium chloride from 0 to 0.5 M. Of the eluted active fractions, a fraction with the highest specific activity was analyzed by SDS-PAGE. The fraction gave a single band. The specific activity of purified enzyme was 120 mU/mg. The purification process is summarized in Table 3.

**[Table 3] Purification of imine reductase from strain GF3546**

| Step | Protein (mg) | Total activity U | Specific activity mU/mg |
|---|---|---|---|
| Cell-free extract | 3,882 | 0.98 | 0.253 |
| Nucleic acid-free | 4,159 | 1.36 | 0.328 |
| Phenyl Sepharose HP | 225.6 | 0.63 | 2.79 |
| MonoQ | 5.94 | 0.71 | 120 |

### [Example 5] Synthesis of (S)-2-methylpyrrolidin with imine reductase

A reaction mixture containing 100 mM potassium phosphate buffer (pH 7.0), 40 mM NADPH, 20 mM 2-methyl-1-pyrroline, and the enzyme prepared as described in Example 4 was incubated at 25°C overnight. Quantity and optical purity of the generated 2-methylpyrrolidine were determined according to Example 3. The result showed that 2-methylpyrrolidine generated by the enzyme was an S isomer of 92.7%ee. The reaction yield was 77.5%.

### [Example 6] Determination of the molecular weight of imine reductase

The enzyme prepared as described in Example 4 was analyzed by SDS-PAGE. The result showed that the subunit molecular weight was about 30,500. Furthermore, the molecular weight determined by gel filtration using Superdex200 column was about 81,500.

### [Example 7] Optimal pH of imine reductase

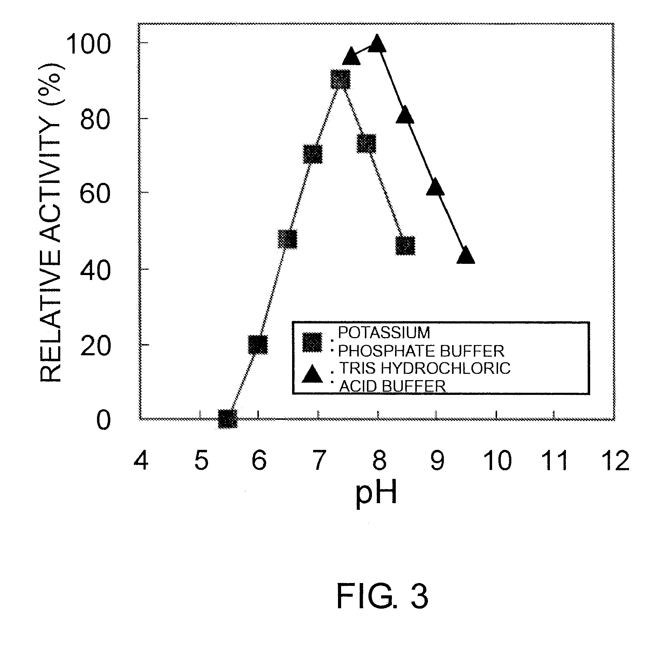

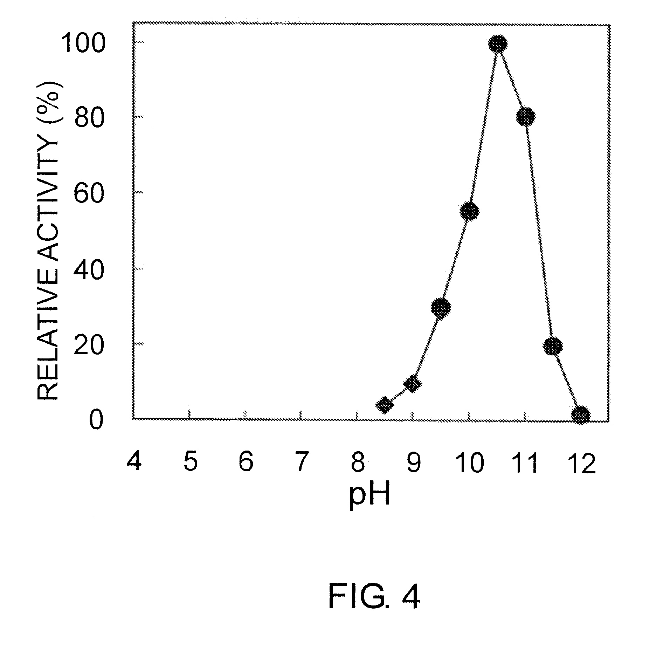

The enzyme prepared as described in Example 3 was assayed for the activity of reducing 2-methyl-1-pyrroline and the activity of oxidizing (S)-2-methylpyrrolidine; by changing the pH using potassium phosphate buffer, Tris-hydrochloric acid buffer, glycine/potassium chloride/potassium hydroxide buffer. With the maximal activity as 100, the relative activities are shown in Figs. 3 and 4. The optimal pH for the reduction was 7.4 to 8.0, and the optimal pH for the oxidation was 10.5. The activity of oxidizing (S)-2-methylpyrrolidine was determined by the standard assay method described below by changing the pH alone.

### Standard assay method for the activity of oxidizing (S)-2-methylpyrrolidine

A reaction mixture containing 100 mM glycine/potassium chloride/potassium hydroxide buffer 10.5, 2.5 mM NADP⁺, 10 mM (S)-2-methylpyrrolidine, and the enzyme was incubated at 30°C. The reaction mixture was assayed to determine the increase in absorbance at 340 nm due to the increase of NADPH. The amount of enzyme that catalyzes the increase of 1 µmol of NADPH per minute was defined as 1 U.

### [Example 8] Optimal temperature of imine reductase

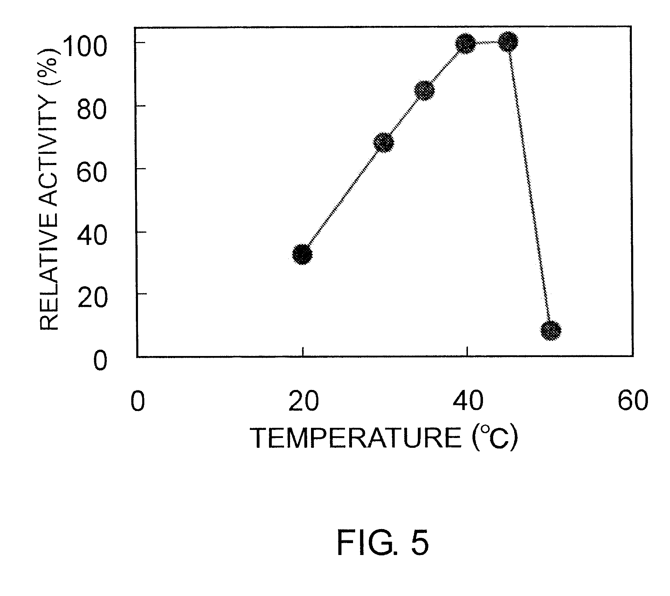

The enzyme prepared as described in Example 4 was assayed for the activity of reducing 2-methyl-1-pyrroline by changing the temperature alone under the standard conditions of imine reduction. With the maximal activity as 100, the relative activity is shown Fig. 5. 80% or more of the maximal activity was achieved in a range of 35 to 45°C.

### [Example 9] Substrate specificity of imine reductase

The enzyme prepared as described in Example 4 was incubated with various imine compounds. With the activity of reducing 2-methyl-1-pyrroline as 100, the relative reducing activity is shown in Table 4.

**[Table 4] Substrate specificity of imine reductase**

| Substrate | Concentration mM | Coenzyme | Relative activity | |
|---|---|---|---|---|
| | | | % | % |
| 2-Methyl-1-pyrroline | 10 | NADPH | 100.0 | |
| 2-Methyl-1-pyrroline | 10 | NADH | 0.0 | |
| N-Benzylidenebenzylamine | 10 | NADPH | 20.9 | |
| N-((E/Z)-N-(1-phenylethylidene)aniline | 10 | NADPH | 5.3 | |
| 1-Methyl-6,7-dimethoxy-3,4-dihydroisoquinoline | 0.05 | NADPH | 8.9 | |
| (S)-2-Methylpyrrolidine | 10 | NADP+ | 44.1 | 100.0 |
| (R)-2-Methylpyrrolidine | 10 | NADP+ | 7.7 | 17.4 |

### [Example 10] Partial amino acid sequence of imine reductase

The N-terminal amino acid sequence of the enzyme prepared as described in Example 9 was analyzed using a protein sequencer. The amino acid sequence is shown in SEQ ID NO: 4. Furthermore, a gel fragment containing the imine reductase was excised from the SDS-PAGE gel. After two washes, trypsin was used to perform in-gel digestion at 35°C overnight. The digested peptide was fractionated by reverse phase HPLC (Tosho; TSK gel ODS-80-Ts, 2.0 mm x 250 mm). The peptide was eluted using 0.1 % trifluoroacetic acid with an acetonitrile gradient and collected.

One of the collected peptide peaks was named T61. An amino acid sequence of the peptide was analyzed with a protein sequencer (Hewlett Packard G1005A Protein Sequencer System). The amino acid sequence of T61 is shown in SEQ ID NO: 5.

### [Example 11] Purification of chromosomal DNA from Streptomyces sp. GF3546

GF3546 was cultured and the microbial cells were harvested according to the method described in Example 3. The chromosomal DNA was purified from the microbial cells according to the method described in the reference of J. Dairy Sci., 75, 1186-1191 (1992).

### [Example 12] Cloning of the imine reductase gene core region

Sense and antisense primers were synthesized based on the N-terminal and T61 amino acid sequences. The nucleotide sequences are shown in SEQ ID NOs: 6 and 7.

Using 50 µl of reaction mixture containing 50 pmol each of the primers, 10 nmol dNTP, 50 ng of chromosomal DNA from *Streptomyces* sp. GF3546, Ex-Taq buffer (Takara Bio), 5% DMSO, and 1 U of Ex-Taq (Takara Bio), PCR was carried out with 30 cycles of denaturation (94°C for 30 seconds), annealing (45°C for 30 seconds), and extension (70°C for one minute and five seconds) in GeneAmp PCR System 9700 (Applied Biosystems).

A part of the PCR reaction mixture was analyzed by agarose gel electrophoresis. A seemingly specific band was detected at around 900 bp. Then, a nucleotide sequence of the DNA fragment was sequenced. The determined nucleotide sequence of the core region is shown in SEQ ID NO: 8.

### [Example 13] Analysis of the nucleotide sequences around the core region of the imine reductase gene

The chromosomal DNA derived from *Streptomyces* sp. GF3546 was digested with restriction enzyme *Sac*I. Each fragment was cyclized via overnight self-ligation at 16°C using T4 ligase. Then, PCR was carried out using 50 µl of reaction mixture containing 100 pmol each of primers shown as SEQ ID NOs: 9 and 10, 25 ng of cyclized DNA, Ex-Taq buffer (Takara Bio), 5% DMSO, and 1 U of Ex-Taq (Takara Bio) with 30 cycles of denaturation (95°C for 30 seconds), annealing (55°C for 30 seconds), and extension (72°C for seven minutes) in GeneAmp PCR System 9700 (Applied Biosystems). A part of each PCR mixture was analyzed by agarose gel electrophoresis. DNA fragment of about 2,000 bp was detected. The nucleotide sequence of DNA fragment was analyzed to determine the ORF sequence of the imine reductase gene. The determined DNA sequence is shown in SEQ ID NO: 2, and the deduced amino acid sequence is shown in SEQ ID NO: 3.

### [Example 14] Cloning of the imine reductase gene

Based on the structural gene sequence for the imine reductase, primers shown as SEQ ID NO: 11 (SsSIR-ATG1) and SEQ ID NO: 12 (SsSIR-TAA1) were synthesized to clone only ORF. PCR was carried out using 50 µl of reaction mixture containing 50 pmol each of the primers, 10 nmol dNTP, 50 ng of chromosomal DNA derived from *Streptomyces* sp. GF3546, *Pfu* Turbo-DNA Polymerase buffer (STRATAGENE), 5% DMSO, and 1.9 U of Pfu Turbo-DNA Polymerase (STRATAGENE) with 30 cycles of denaturation (98°C for 30 seconds), annealing (60°C for one minute), and extension (72°C for one minute and 10 seconds) in GeneAmp PCR System 9700 (Applied Biosystems).

The resulting DNA fragment was purified and collected using GFX PCR DNA and the Gel Band Purification kit (GE Healthcare). The DNA fragment was double-digested with restriction enzymes *Bsp*HI and *Xba*I. After agarose gel electrophoresis, a portion containing a band of interest was excised and the DNA fragment was purified using GFX PCR DNA and the Gel Band Purification kit (GE Healthcare).

Using the Takara Ligation kit, the prepared DNA fragment was ligated to pSE420D (a plasmid constructed by modifying the multicloning site of plasmid vector pSE420 from Invitrogen; JP-A (Kokai) 2000-189170) double-digested with *Nco*I and *Xba*I. The *E. coli* JM109 strain was transformed with the resulting vector.

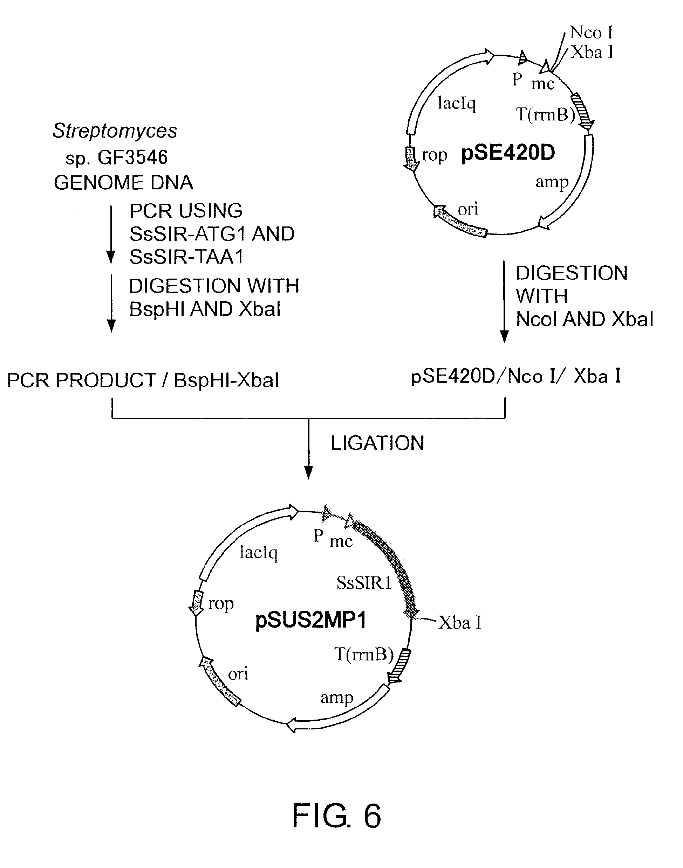

The transformant was grown on an ampicillin-containing LB medium. The plasmid was extracted from the grown transformant. The plasmid was named pSUS2MP1 and confirmed to have the imine reductase gene as an insert. The process of plasmid construction is shown in Fig. 6.

### [Example 15] Assessment of the activity of recombinant imine reductase

The *E. coli* JM109 strain that had been transformed with an imine reductase expression plasmid pSUS2MP1 was cultured in an ampicillin-containing liquid LB medium at 30°C overnight. After adding 0.1 mM IPTG, the cells were further cultured for four hours.

The microbial cells were harvested by centrifugation, and then suspended in 100 mM potassium phosphate buffer (pH 7.0) containing 1 mM DTT. The microbial cells were crushed in Sealed-Type Ultrasonic Cell Disruptor UCD-200TM (Cosmo Bio). After centrifugation, the supernatant was collected as microbial cell extract. Meanwhile, *E. coli* JM109 that does not contain the gene was cultured in LB medium overnight. After adding 0.1 mM IPTG, the cells were further cultured for four hours. Using the same method, the microbial cells were also crushed to prepare microbial cell extract.

Each microbial cell extract was assayed for its imine-reducing activity towards 2-methyl-1-pyrroline. The result is shown in Table 5. The method for assaying the activity was the same as described in Example 4.

**[Table 5]**

| Plasmid | Imine-reducing activity mU/mg | Glucose dehydrogenase activity U/mg |
|---|---|---|
| None | 0 | 0 |
| pSUS2MP1 | 5.91 | 0 |
| pSG-S2MP | 5.35 | 12.1 |

### [Example 16] Construction of plasmid pSG-S2MP for coexpressing the imine reductase and Bacillus-derived glucose dehydrogenase

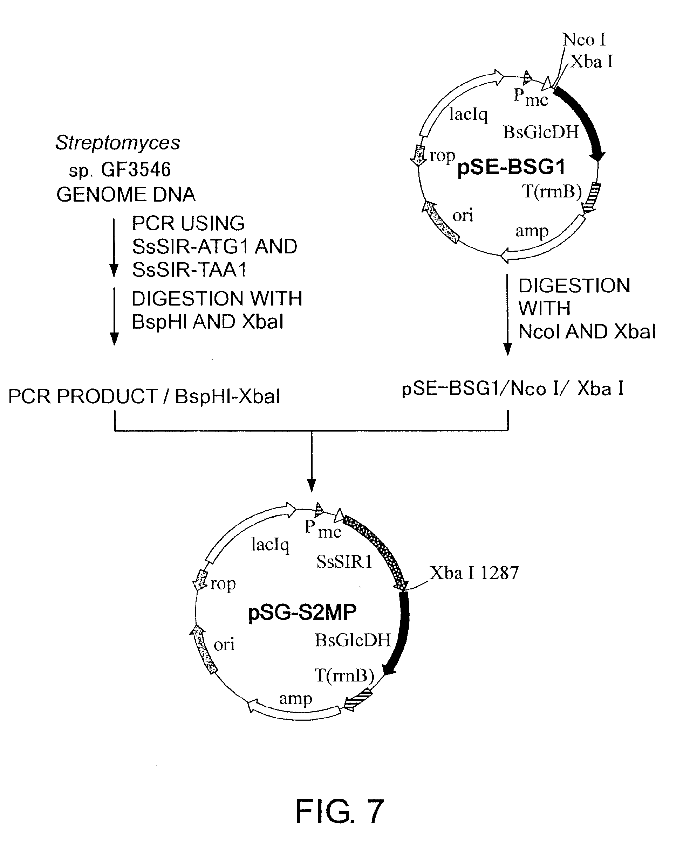

PCR was conducted similarly to Example 14. Amplified DNA fragment containing imine reductase was double-digested with *Bsp*HI and *Xba*I. Then, plasmid pSE-BSG1 (JP-A (Kokai) 2000-189170), which express a *Bacillus*-derived glucose dehydrogenase gene, was double-digested with restriction enzymes *Nco*I and *Xba*I. A DNA fragment containing the imine reductase gene was ligated to the digested pSE-BSG1 using the Takara Ligation kit to construct the plasmid pSG-S2MP which can be used to coexpress imine reductase and glucose dehydrogenase. The process of plasmid construction is shown in Fig. 7.

### [Example 17] Coexpression of imine reductase and glucose dehydrogenase in E. coli

The *E. coli* JM109 strain that had been transformed with pSG-S2MP was cultured in an ampicillin-containing liquid LB medium at 30°C overnight. After adding 0.1 mM IPTG, the cells were further cultured for four hours.

The microbial cells were harvested by centrifugation, and then suspended in 100 mM potassium phosphate buffer (pH 7.0) containing 1 mM DTT. The microbial cells were crushed in Sealed-Type Ultrasonic Cell Disruptor UCD-200TM (Cosmo Bio). The microbial cell lysate was centrifuged, and the supernatant was collected as microbial cell extract. The extract was assayed for its imine-reducing activity towards 2-methyl-1-pyrroline and its glucose dehydrogenase activity. The result is shown in Table 5. The glucose dehydrogenase activity was assayed at 30°C using a reaction solution containing 100 mM potassium phosphate buffer (pH 6.5), 2.5 mM NADP⁺, 100 mM glucose, and microbial cell extract. The amount of enzyme that catalyzes the generation of 1 µmol of NADPH per minute under the-above described conditions was defined as 1 U.

### [Example 18] Production of (S)-2-methylpyrrolidine by recombinant E. coli

The *E. coli* JM109 strain that had been transformed with pSG-S2MP was cultured in an ampicillin-containing liquid LB medium (5 ml) at 30°C overnight. After adding 0.1 mM IPTG, the cells were further cultured for four hours.

The microbial cells were harvested by centrifugation, and then suspended in 1 ml of 100 mM potassium phosphate buffer (pH 7.0) containing 50 mM 2-methyl-1-pyrroline and 100 mM glucose. The suspension was reacted at 25°C while shaking overnight. After the reaction, the reaction mixture was assayed to determine the optical purity of the generated 2-methylpyrrolidine, and the concentrations of 2-methyl-1-pyrroline and 2-methylpyrrolidine in the reaction mixture by the same method as described in Example 8. The optical purity of the generated (S)-2-methylpyrrolidine was 92.0%ee.

### Industrial Applicability

The present invention provides methods for efficiently producing optically active amine derivatives by stereoselective reduction using the microorganisms' reduction ability. The methods of the present invention are industrially advantageous because high stereoselectivity can be expected. Optically active amine derivatives produced by the present invention are useful as materials for optically active pharmaceutical agents. For example, (S)-2-methylpyrrolidine is a useful compound as a material for pharmaceutical agents.

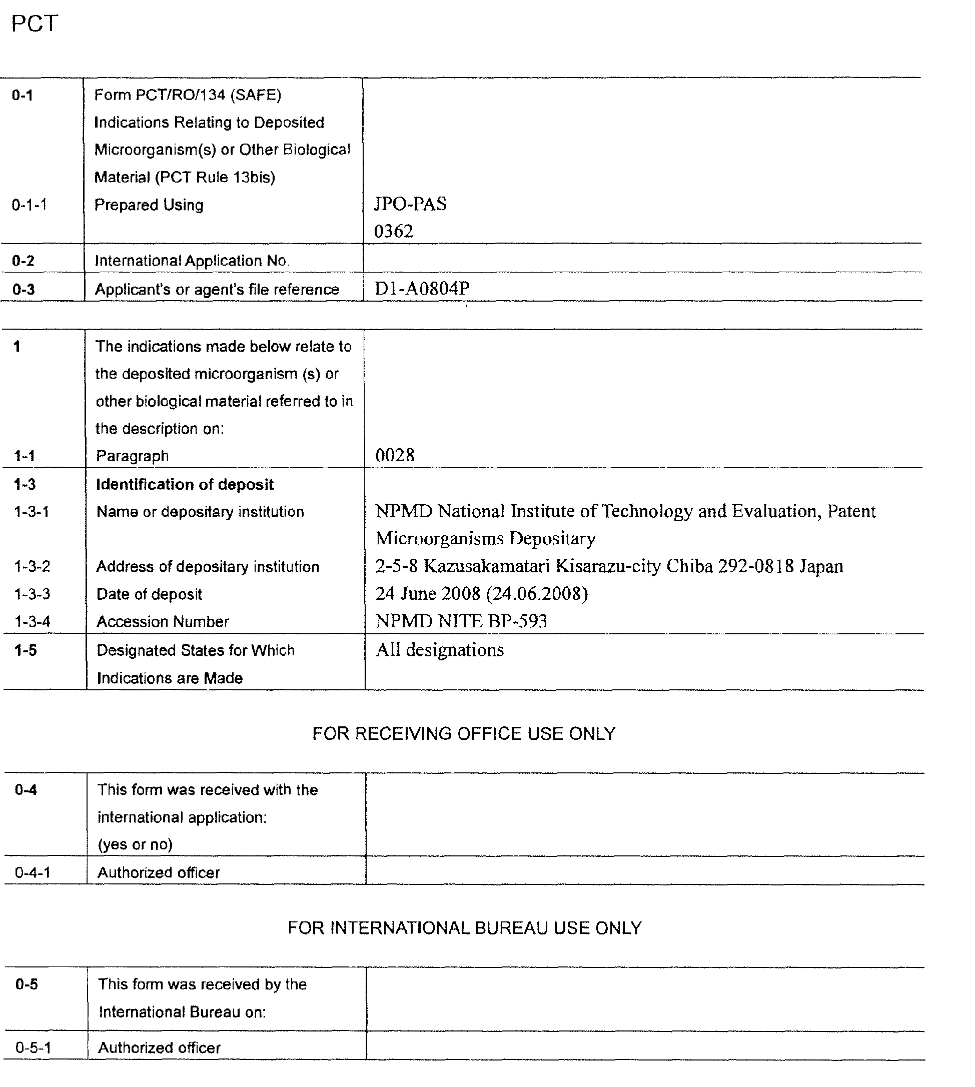

## Claims

1. An imine reductase having the physicochemical properties of (1) to (5) below:
(1) activity: to generate (S)-2-methylpyrrolidine by reducing 2-methyl-1-pyrroline in a reduced nicotinamide adenine dinucleotide phosphate (hereinafter abbreviated as NADPH)-dependent manner and generate 2-methyl-1-pyrroline by oxidizing (S)-2-methylpyrrolidine in a nicotinamide adenine dinucleotide phosphate (hereinafter abbreviated as NADP⁺)-dependent manner;
(2) coenzyme dependency: use NADPH as a coenzyme for reduction and use NADP⁺ as a coenzyme for oxidation;
(3) optimal pH: 7.4 to 8.0 for reduction and pH 10.5 for oxidation;
(4) optimal temperature: 35 to 45°C for reduction; and
(5) molecular weight: approximately 30,500 by sodium dodecyl sulfate-polyacrylamide gel electrophoresis (hereinafter abbreviated as SDS-PAGE) and approximately 81,500 by gel filtration.

2. An imine reductase that has the physicochemical properties of (1) and (2) of claim 1, and which is encoded by the polynucleotide of any one of (a) to (e) below:
(a) a polynucleotide comprising the nucleotide sequence of SEQ ID NO: 2;
(b) a polynucleotide encoding a protein comprising the amino acid sequence of SEQ ID NO: 3;
(c) a polynucleotide encoding a protein comprising an amino acid sequence with a substitution, deletion, insertion, and/or addition of one or more amino acids in the amino acid sequence of SEQ ID NO: 3;
(d) a polynucleotide that hybridizes under a stringent condition to a DNA comprising the nucleotide sequence of SEQ ID NO: 2; and
(e) a polynucleotide encoding an amino acid sequence with 80% or higher sequence identity to the amino acid sequence of SEQ ID NO: 3.

3. A method for producing an optically active S-isomer amine derivative, which comprises the steps of:
contacting an imine derivative represented by formula (I) with at least one enzymatically active material selected from the group consisting of: the imine reductase of claim 1 or 2, a microbial cell that produces the imine reductase, and a processed product thereof; and
collecting the optically active S-isomer amine derivative represented by formula (II): 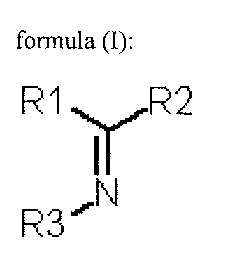 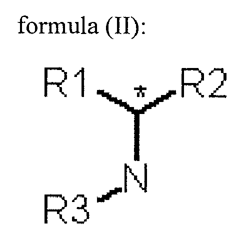 (wherein R1 and R2 groups each represents an alkyl group having one to three carbon atoms; R3 group represents a hydrogen or an alkyl group having one to three carbon atoms; and R1 and R3 together may form a ring).

4. A method for producing an optically active S-isomer amine derivative, which comprises the steps of:
contacting an imine derivative represented by formula (I) with at least one enzymatically active material selected from the group consisting of: a culture of microorganism having the ability to stereoselectively reduce the compound, a microbial cell, and a processed product thereof; and
collecting the optically active S-isomer amine derivative represented by formula (II): 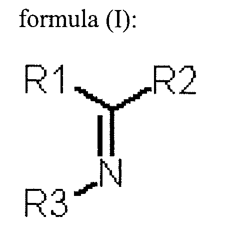 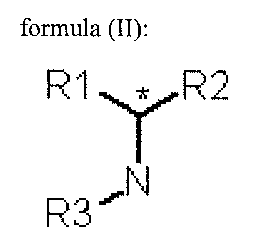 (wherein R1 and R2 groups each represents an alkyl group having one to three carbon atoms; R3 group represents a hydrogen or an alkyl group having one to three carbon atoms; and R1 and R3 together may form a ring).

5. The method of claim 4, wherein the compound of formula (I) is an imine derivative represented by formula (III) below and the compound of formula (II) is an amine derivative represented by formula (IV): 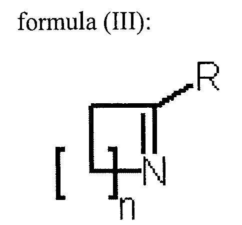 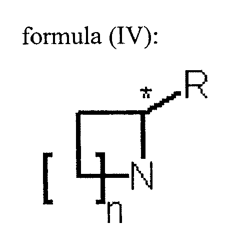 (wherein R group represents an alkyl group having one to three carbon atoms; and n represents an integer of 1 to 4).

6. The method of claim 5 for producing an optically active S-isomer amine derivative, wherein the compound represented by formula (III) is 2-methyl-1-pyrroline and the compound represented by formula (IV) is 2-methylpyrrolidine.

7. The method of any one of claims 4 to 6 for producing an optically active S-isomer amine derivative, wherein the microorganism belongs to the genus *Streptomyces*.

8. The method of any one of claims 4 to 6 for producing an optically active S-isomer amine derivative, wherein the microorganism is *Streptomyces* sp.

9. The method of any one of claims 4 to 6 for producing an optically active S-isomer amine derivative, wherein the microorganism is *Streptomyces* sp. NITE P-593.

10. A polynucleotide encoding an imine reductase that has the physicochemical properties of (1) and (2) of claim 1, which is any one of the following polynucleotides:
(a) a polynucleotide comprising the nucleotide sequence of SEQ ID NO: 2;
(b) a polynucleotide encoding a protein comprising the amino acid sequence of SEQ ID NO: 3;
(c) a polynucleotide encoding a protein comprising an amino acid sequence with a substitution, deletion, insertion, and/or addition of one or more amino acids in the amino acid sequence of SEQ ID NO: 3;
(d) a polynucleotide that hybridizes under a stringent condition to a DNA comprising the nucleotide sequence of SEQ ID NO: 2; and
(e) a polynucleotide encoding an amino acid sequence with 80% or higher sequence identity to the amino acid sequence of SEQ ID NO: 3.

11. A recombinant vector inserted with the polynucleotide of claim 10.

12. The recombinant vector of claim 11, which is inserted additionally with a polynucleotide encoding a dehydrogenase that is capable of catalyzing a redox reaction using NADP⁺ as a coenzyme.

13. The vector of claim 12, wherein the dehydrogenase is a glucose dehydrogenase.

14. The recombinant vector of claim 12, wherein the glucose dehydrogenase is derived from *Bacillus subtilis.*

15. A transformant that maintains the polynucleotide of claim 10 or the vector of claim 12 in an expressible manner.

16. A method for producing a protein encoded by the polynucleotide of claim 10, which comprises the step of culturing the transformant of claim 15 and collecting an expression product.
